(19) Europäisches Patentamt
European Patent Office
Office européen des brevets

(11) EP 2 947 090 B1

(12) **EUROPÄISCHE PATENTSCHRIFT**

(45) Veröffentlichungstag und Bekanntmachung des Hinweises auf die Patenterteilung:
**24.08.2016 Patentblatt 2016/34**

(51) Int Cl.:
***C07F 9/6574*** *(2006.01)* ***C07F 9/145*** *(2006.01)*
***B01J 31/18*** *(2006.01)* ***C07C 45/50*** *(2006.01)*

(21) Anmeldenummer: **15166027.1**

(22) Anmeldetag: **30.04.2015**

(54) **NEUE MONOPHOSPHITLIGANDEN MIT EINER TERT-BUTYLOXYCARBONYL-GRUPPE**

NOVEL MONOPHOSPHITE LIGANDS WITH A TERTIARY BUTYLOXYCARBONYL GROUP

NOUVEAUX LIGANDS DE MONO-PHOSPHITE AYANT UN GROUPE TERT-BUTYLOXYCARBONYLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.05.2014 DE 102014209532**

(43) Veröffentlichungstag der Anmeldung:
**25.11.2015 Patentblatt 2015/48**

(73) Patentinhaber: **Evonik Degussa GmbH**
**45128 Essen (DE)**

(72) Erfinder:
- **Dyballa, Katrin Marie**
**45657 Recklinghausen (DE)**
- **Franke, Robert**
**45772 Marl (DE)**
- **Hess, Dieter**
**45770 Marl (DE)**
- **Fridag, Dirk**
**45721 Haltern am See (DE)**
- **Geilen, Frank**
**45721 Haltern am See (DE)**

(56) Entgegenhaltungen:
**EP-A2- 0 614 870**
**WO-A1-2010/057099**
**DE-A1-102011 085 883**

- **RUI M B CARRILHO ET AL: "Rhodium/tris-binaphthyl chiral monophosphite complexes: Efficient catalysts for the hydroformylation of disubstituted aryl olefins", JOURNAL OF ORGANOMETALLIC CHEMISTRY, ELSEVIER-SEQUOIA S.A. LAUSANNE, CH, Bd. 698, 5. Oktober 2011 (2011-10-05), Seiten 28-34, XP028392107, ISSN: 0022-328X, DOI: 10.1016/J.JORGANCHEM.2011.10.007 [gefunden am 2011-10-29]**
- **KONSTANTIN N GAVRILOV ET AL: "Phosphites and diamidophosphites based on mono-ethers of BINOL: a comparison of enantioselectivity in asymmetric catalytic reactions", TETRAHEDRON, ELSEVIER SCIENCE PUBLISHERS, AMSTERDAM, NL, Bd. 68, Nr. 5, 28. November 2011 (2011-11-28), Seiten 1581-1589, XP028436933, ISSN: 0040-4020, DOI: 10.1016/J.TET.2011.11.092 [gefunden am 2011-12-06]**
- **ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803**
- **ANA Z. GONZÁLEZ ET AL: "Gold(I)-Catalyzed Enantioselective [4 + 2]-Cycloaddition of Allene-dienes", ORGANIC LETTERS, Bd. 12, Nr. 1, 1. Januar 2010 (2010-01-01) , Seiten 200-203, XP055201269, ISSN: 1523-7060, DOI: 10.1021/ol902622b**

EP 2 947 090 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft neue Monophosphitliganden mit einer *tert*-Butyloxycarbonyl-Gruppe (BOC-Gruppe), deren Verwendung als Liganden in der Hydroformylierung und das Hydroformylierungsverfahren.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor $P^{III}$. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

In Angew. Chem. Int. Ed. 2000, 39, No. 9, S.1639-1641 von Börner et al. werden Liganden beschrieben, welche eine P-C- und zwei P-O-Bindungen aufweisen, es handelt sich somit um Phosphonite. Die dort beschriebenen Phosphonite weisen bei einem Einsatz in der Hydroformylierung n/iso-Selektivitäten (n/iso = das Verhältnis von linearem Aldehyd (= n) zu verzweigtem (= iso) Aldehyd)) von 0,61 bis 1,57 auf.

Die in DE 199 54 721 beschriebenen Phosphonitliganden weisen eine gute n/iso-Selektivität auf. Allerdings haben eigene Untersuchungen ergeben, dass die Verbindung II-c (in DE 199 54 721; Seite 6) zu einer photochemisch induzierten Zersetzung neigt, weshalb von einem großtechnischen Einsatz abzusehen ist.

Ein Nachteil der Liganden mit einer Phosphonitstruktur besteht in der sehr aufwändigen Herstellung. Die Möglichkeit einer günstigen und einfachen Synthese spielt jedoch für den Einsatz von Liganden in einem großtechnischen Prozess aber eine elementare Rolle.

Die einfache Verfügbarkeit und damit verbunden die gute Möglichkeit eines großtechnischen Einsatzes ist ein wichtiges Kriterium, da der Herstellungsaufwand und damit verbunden die anfallenden Produktionskosten für den Liganden nur so hoch sein dürfen, dass die Rentabilität des Gesamtprozesses, in dem der Ligand später eingesetzt wird, weiterhin gewährleistet ist.

Rhodium-Monophosphit-Komplexe in katalytisch aktiven Zusammensetzungen sind geeignet für die Hydroformylierung von verzweigten Olefinen mit innenständigen Doppelbindungen.

Seit den 1970er Jahren ist die Verwendung von so genannten "bulky phosphites" in der Hydroformylierung beschrieben (siehe u.a. van Leeuwen et. al, Journal of Catalysis, 2013, 298, 198-205). Diese zeichnen sich durch eine gute Aktivität aus, jedoch ist die n/i-Selektivität für endständig oxierte Verbindungen verbesserungswürdig.

Aus EP 0 155 508 ist die Verwendung von bisarylensubstituierten Monophosphiten bei der rhodiumkatalysierten Hydroformylierung von sterisch gehinderten Olefinen, z. B. Isobuten, bekannt. Hierbei werden jedoch z.T. sehr hohe Rhodiumkonzentrationen verwendet (u.a. 250 ppm), was in Anbetracht des derzeitigen Rhodiumpreises für ein großtechnisches Verfahren inakzeptabel ist und verbessert werden muss.

Für Hydroformylierungsreaktionen ist derzeit Tris(2,4-di-tert-butylphenyl)phosphit (TDTBPP) einer der leistungsstärksten und kommerziell verfügbaren Monophosphitliganden, welcher unter dem Handelsnamen Alkanox 240 erhältlich ist (siehe auch R. Franke, D. Selent, A. Börner, "Applied Hydroformylation", Chem. Rev., 2012, 112, S.5681 Kapitel 3.4.2).

Die technische Aufgabe, welche der vorliegenden Erfindung zugrunde lag, ist die Bereitstellung eines neuen Monophosphitliganden, welcher in der Hydroformylierung von ungesättigten Verbindungen nicht die aus dem Stand der Technik zuvor aufgezeigten Nachteile aufweist.

Zum einen soll der Herstellungsaufwand geringer sein als der bei den oben beschriebenen Phosphonitliganden, zum anderen soll eine gute n-Selektivität in Bezug auf die Hydroformylierung erzielt werden.

Insbesondere sollen die neuen Monophosphitliganden auch zur Oxierung von technischen Olefingemischen, die hauptsächlich verzweigte Olefine mit innenständigen Doppelbindungen enthalten, geeignet sein. Bei der Oxierung soll hierbei ein hoher Anteil an gewünschten endständig hydroformylierten Produkten erhalten werden.

Die Aufgabe wird gelöst durch einen Liganden nach Anspruch 1.

Ligand, welcher eine der beiden allgemeinen Strukturen **I** oder **II** aufweist:

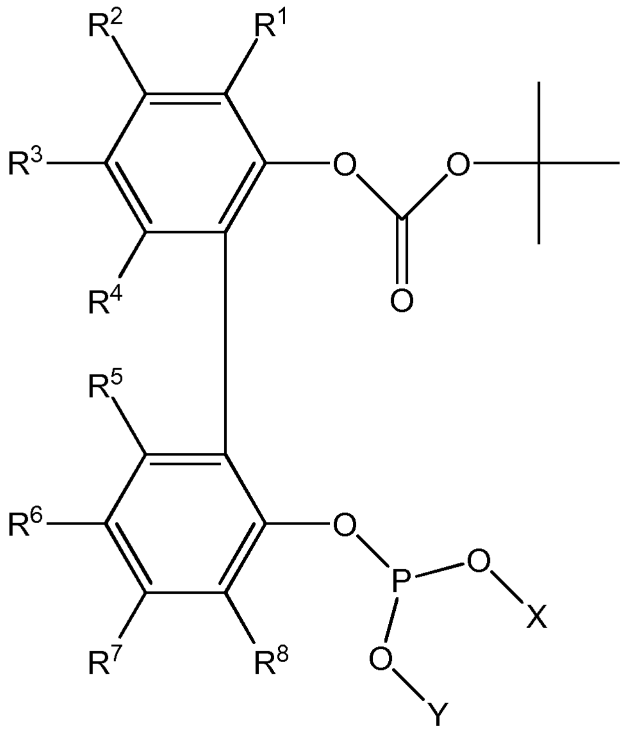

I

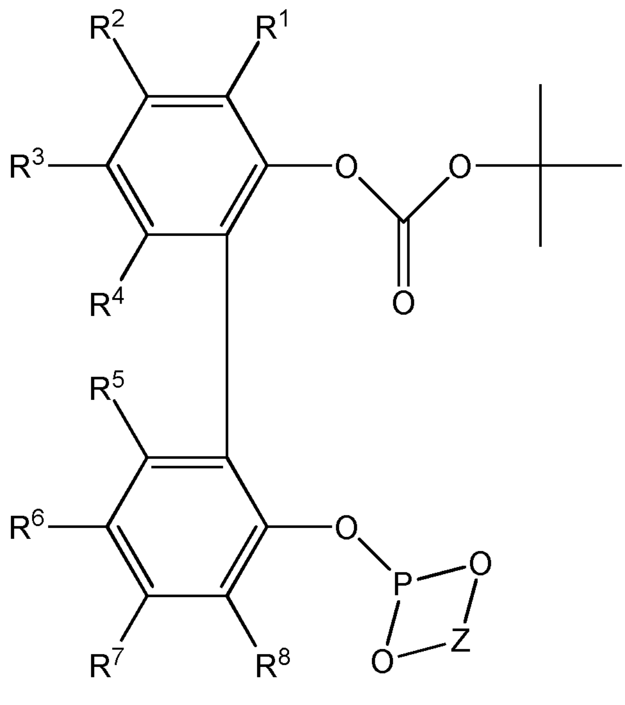

II

wobei

$R^1$, $R^2$, $R^3$, $R^4$, $R^5$, $R^6$, $R^7$, $R^8$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, Halogen (wie Cl, F, Br, I), COO-($C_1$-$C_{12}$)-Alkyl, CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl,-CO-($C_6$-$C_{20}$)-Aryl,-COOH, -OH, -$SO_3H$; -$SO_3Na$, -$NO_2$, -CN, -$NH_2$;

-N[$(C_1$-$C_{12})$-Alkyl]$_2$;
X und Y jeweils unabhängig voneinander ausgewählt sind aus:

-$(C_1$-$C_{12})$-Alkyl, -$(C_6$-$C_{20})$-Aryl, -$(C_6$-$C_{20})$-Aryl-$(C_1$-$C_{12})$-Alkyl, -$(C_6$-$C_{20})$-Aryl-O-$(C_1$-$C_{12})$-Alkyl, -$(C_1$-$C_{12})$-Alkyl-$(C_6$-$C_{20})$-Aryl, -$(C_6$-$C_{20})$-Aryl-COO-$(C_1$-$C_{12})$-Alkyl, -$(C_6$-$C_{20})$-Aryl-CONH-$(C_1$-$C_{12})$-Alkyl,-$(C_6$-$C_{20})$-Aryl-CON[$(C_1$-$C_{12})$-Alkyl]$_2$, -$(C_4$-$C_{20})$-Heteroaryl, -$(C_4$-$C_{20})$-Heteroaryl-$(C_1$-$C_{12})$-Alkyl,-$(C_5$-$C_8)$-Cycloalkyl, -$(C_6$-$C_{20})$-Aryl-CO-$(C_6$-$C_{20})$-Aryl,

Z ausgewählt ist aus:

-$(C_1$-$C_{12})$-Alkyl-, -$(C_6$-$C_{20})$-Aryl-, -$(C_6$-$C_{20})$-Aryl-$(C_1$-$C_{12})$-Alkyl-, -$(C_1$-$C_{12})$-Alkyl-$(C_6$-$C_{20})$-Aryl-, -$(C_4$-$C_{20})$-Heteroaryl-, -$(C_6$-$C_{20})$-Aryl-CO-$(C_6$-$C_{20})$-Aryl-, -$(C_6$-$C_{20})$-Aryl-$(C_6$-$C_{20})$-Aryl-;

wobei die genannten Alkyl-, Heteroalkyl-, Cycloalkyl-, Heterocycloalkyl-, Aryl- und Heteroarylgruppen substituiert sein können.

$(C_1$-$C_{12})$-Alkyl und O-$(C_1$-$C_{12})$-Alkyl können jeweils unsubstituiert oder durch einen oder mehrere gleiche oder verschiedene Reste substituiert sein, die ausgewählt sind unter $(C_3$-$C_{12})$-Cycloalkyl, $(C_3$-$C_{12})$-Heterocycloalkyl, $(C_6$-$C_{20})$-Aryl, Fluor, Chlor, Cyano, Formyl, Acyl oder Alkoxycarbonyl.

$(C_3$-$C_{12})$-Cycloalkyl und $(C_3$-$C_{12})$-Heterocycloalkyl können jeweils unsubstituiert oder durch einen oder mehrere gleiche oder verschiedene Reste substituiert sein, die ausgewählt sind unter $(C_1$-$C_{12})$-Alkyl, $(C_1$-$C_{12})$-Alkoxy, $(C_3$-$C_{12})$-Cycloalkyl, $(C_3$-$C_{12})$-Heterocycloalkyl, $(C_6$-$C_{20})$-Aryl, Fluor, Chlor, Cyano, Formyl, Acyl oder Alkoxycarbonyl.

$(C_6$-$C_{20})$-Aryl und -$(C_6$-$C_{20})$-Aryl-$(C_6$-$C_{20})$-Aryl- können jeweils unsubstituiert oder durch einen oder mehrere gleiche oder verschiedene Reste substituiert sein, die ausgewählt sind unter -H, -$(C_1$-$C_{12})$-Alkyl, -O-$(C_1$-$C_{12})$-Alkyl, -O-$(C_6$-$C_{20})$-Aryl, -$(C_6$-$C_{20})$-Aryl, -Halogen (wie Cl, F, Br, I), -COO-$(C_1$-$C_{12})$-Alkyl, -CONH-$(C_1$-$C_{12})$-Alkyl, -$(C_6$-$C_{20})$-Aryl-CON[$(C_1$-$C_{12})$-Alkyl]$_2$, -CO-$(C_1$-$C_{12})$-Alkyl, -CO-$(C_6$-$C_{20})$-Aryl, -COOH, -OH, -$SO_3H$; -$SO_3Na$, -$NO_2$, -CN, -$NH_2$, -N[$(C_1$-$C_{12})$-Alkyl]$_2$.

Im Rahmen der Erfindung umfasst der Ausdruck -$(C_1$-$C_{12})$-Alkyl geradkettige und verzweigte Alkylgruppen. Vorzugsweise handelt es sich dabei um unsubstituierte geradkettige oder verzweigte -$(C_1$-$C_8)$-Alkyl- und ganz bevorzugt -$(C_1$-$C_6)$-Alkylgruppen. Beispiele für -$(C_1$-$C_{12})$-Alkylgruppen sind insbesondere Methyl, Ethyl, Propyl, Isopropyl, n-Butyl, ido-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -$(C_1$-$C_{12})$-Alkyl gelten auch für die Alkylgruppen in -O-$(C_1$-$C_{12})$-Alkyl, also in -$(C_1$-$C_{12})$-Alkoxy. Vorzugsweise handelt es sich dabei um unsubstituierte geradkettige oder verzweigte -$(C_1$-$C_6)$-Alkoxygruppen.

Substituierte -$(C_1$-$C_{12})$-Alkylgruppen und substituierte -$(C_1$-$C_{12})$-Alkoxygruppen können in Abhängigkeit von ihrer Kettenlänge, einen oder mehrere Substituenten aufweisen. Die Substituenten sind vorzugsweise unabhängig voneinander ausgewählt unter -$(C_3$-$C_{12})$-Cycloalkyl, -$(C_3$-$C_{12})$-Heterocycloalkyl, -$(C_6$-$C_{20})$-Aryl, Fluor, Chlor, Cyano, Formyl, Acyl oder Alkoxycarbonyl,

Der Ausdruck -$(C_3$-$C_{12})$-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Der Ausdruck -$(C_3$-$C_{12})$-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -$(C_3$-$C_{12})$-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1,2,3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -$(C_3$-$C_{12})$-Heterocycloalkylgruppen Tetrahydrothiophenyl, Tetrhydrofuryl, Tetrahydropyranyl und Dioxanyl.

Substituierte -$(C_3$-$C_{12})$-Cycloalkylgruppen und substituierte -$(C_3$-$C_{12})$-Heterocycloalkylgruppen können, in Abhängigkeit von ihrer Ringgröße, einen oder mehrere (z.B. 1, 2, 3, 4 oder 5) weitere Substituenten aufweisen. Diese Substituenten sind vorzugsweise unabhängig voneinander ausgewählt unter -$(C_1$-$C_{12})$-Alkyl, -$(C_1$-$C_{12})$-Alkoxy, -$(C_3$-$C_{12})$-Cycloalkyl, -$(C_3$-$C_{12})$-Heterocycloalkyl, -$(C_6$-$C_{20})$-Aryl, Fluor, Chlor, Cyano, Formyl, Acyl oder Alkoxycarbonyl. Substituierte -$(C_3$-$C_{12})$-Cycloalkylgruppen tragen vorzugsweise eine oder mehrere -$(C_1$-$C_6)$-Alkylgruppen. Substituierte -$(C_3$-$C_{12})$-Heterocycloalkylgruppen tragen vorzugsweise eine oder mehrere -$(C_1$-$C_6)$-Alkylgruppen.

Der Ausdruck -$(C_6$-$C_{20})$-Aryl und -$(C_6$-$C_{20})$-Aryl-$(C_6$-$C_{20})$-Aryl- umfasst im Sinne der vorliegenden Erfindung

mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -($C_6$-$C_{10}$)-Aryl und -($C_6$-$C_{10}$)-Aryl-($C_6$-$C_{10}$)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -($C_6$-$C_{20}$)-Arylgruppen und -($C_6$-$C_{20}$)-Aryl-($C_6$-$C_{20}$)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I),-COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3H$; -$SO_3Na$, -$NO_2$, -CN, -$NH_2$, -N[($C_1$-$C_{12}$)-Alkyl]$_2$.

Substituierte -($C_6$-$C_{20}$)-Arylgruppen und -($C_6$-$C_{20}$)-Aryl-($C_6$-$C_{20}$)-Arylgruppen sind vorzugsweise substituierte -($C_6$-$C_{10}$)-Arylgruppen und -($C_6$-$C_{10}$)-Aryl-($C_6$-$C_{10}$)-Arylgruppen, insbesondere substituiertes Phenyl oder substituiertes Naphthyl oder substituiertes Anthracenyl. Substituierte -($C_6$-$C_{20}$)-Arylgruppen tragen vorzugsweise eine oder mehrere z.B. 1, 2, 3, 4 oder 5 Substituenten, ausgewählt unter -($C_1$-$C_{12}$)-Alkylgruppen, -($C_1$-$C_{12}$)-Alkoxygruppen.

In einer Ausführungsform sind X und Y jeweils unabhängig voneinander ausgewählt aus:

-($C_1$-$C_{12}$)-Alkyl, -($C_6$-$C_{20}$)-Aryl, -($C_6$-$C_{20}$)-Aryl-($C_1$-$C_{12}$)-Alkyl, -($C_6$-$C_{20}$)-Aryl-O-($C_1$-$C_{12}$)-Alkyl, -($C_6$-$C_{20}$)-Aryl-COO-($C_1$-$C_{12}$)-Alkyl, -($C_6$-$C_{20}$)-Aryl-CONH-($C_1$-$C_{12}$)-Alkyl, -($C_6$-$C_{20}$)-Aryl-CON[($C_1$-$C_{12}$)-Alkyl]$_2$, -($C_4$-$C_{20}$)-Heteroaryl, -($C_4$-$C_{20}$)-Heteroaryl-($C_1$-$C_{12}$)-Alkyl.

In einer Ausführungsform sind X und Y jeweils unabhängig voneinander ausgewählt aus:

-($C_1$-$C_{12}$)-Alkyl, -($C_6$-$C_{20}$)-Aryl, -($C_6$-$C_{20}$)-Aryl-($C_1$-$C_{12}$)-Alkyl, -($C_6$-$C_{20}$)-Aryl-O-($C_1$-$C_{12}$)-Alkyl, -($C_6$-$C_{20}$)-Aryl-COO-($C_1$-$C_{12}$)-Alkyl.

In einer Ausführungsform sind X und Y jeweils unabhängig voneinander ausgewählt aus:

-($C_1$-$C_{12}$)-Alkyl, -($C_6$-$C_{20}$)-Aryl, -($C_6$-$C_{20}$)-Aryl-($C_1$-$C_{12}$)-Alkyl, -($C_6$-$C_{20}$)-Aryl-O-($C_1$-$C_{12}$)-Alkyl.

In einer Ausführungsform ist Z ausgewählt aus:

-($C_1$-$C_{12}$)-Alkyl-, -($C_6$-$C_{20}$)-Aryl-, -($C_6$-$C_{20}$)-Aryl-($C_1$-$C_{12}$)-Alkyl-, -($C_6$-$C_{20}$)-Aryl-CO-($C_6$-$C_{20}$)-Aryl-,-($C_1$-$C_{12}$)-Alkyl-($C_6$-$C_{20}$)-Aryl-, -($C_6$-$C_{20}$)-Aryl-($C_6$-$C_{20}$)-Aryl-.

In einer Ausführungsform steht Z für:

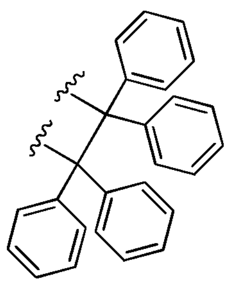

In einer Ausführungsform steht Z für:

-($C_6$-$C_{20}$)-Aryl-($C_6$-$C_{20}$)-Aryl-.

In einer Ausführungsform sind $R^1$, $R^2$, $R^3$, $R^4$, $R^5$, $R^6$, $R^7$, $R^8$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl, - CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$NH_2$, -N[($C_1$-$C_{12}$)-Alkyl]$_2$.

In einer Ausführungsform sind $R^1$, $R^2$, $R^3$, $R^4$, $R^5$, $R^6$, $R^7$, $R^8$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl,-N[($C_1$-$C_{12}$)-Alkyl]$_2$.

In einer Ausführungsform sind $R^1$, $R^2$, $R^3$, $R^4$, $R^5$, $R^6$, $R^7$, $R^8$ jeweils unabhängig voneinander ausgewählt aus:

-H, -($C_1$-$C_{12}$)-Alkyl, -O-($C_1$-$C_{12}$)-Alkyl, -($C_6$-$C_{20}$)-Aryl.

In einer Ausführungsform stehen X und Y für die gleichen Reste.

In einer Ausführungsform stehen $R^3$ und $R^6$ für -O-($C_1$-$C_{12}$)-Alkyl.

In einer Ausführungsform stehen $R^3$ und $R^6$ für -OMe.

In einer Ausführungsform stehen $R^1$ und $R^8$ für -($C_1$-$C_{12}$)-Alkyl.

In einer Ausführungsform stehen $R^1$ und $R^8$ für *tert*-Butyl.

In einer Ausführungsform stehen $R^1$, $R^3$, $R^6$ und $R^8$ für -($C_1$-$C_{12}$)-Alkyl.

In einer Ausführungsform stehen $R^1$, $R^3$, $R^6$ und $R^8$ für Methyl.

In einer Ausführungsform stehen $R^1$, $R^3$, $R^6$ und $R^8$ für *tert*-Butyl.

In einer Ausführungsform stehen sind $R^1$, $R^2$, $R^3$, $R^4$, $R^5$, $R^6$, $R^7$ und $R^8$ für -H.

In einer Ausführungsform weist der Ligand die allgemeine Strukturen **III** auf:

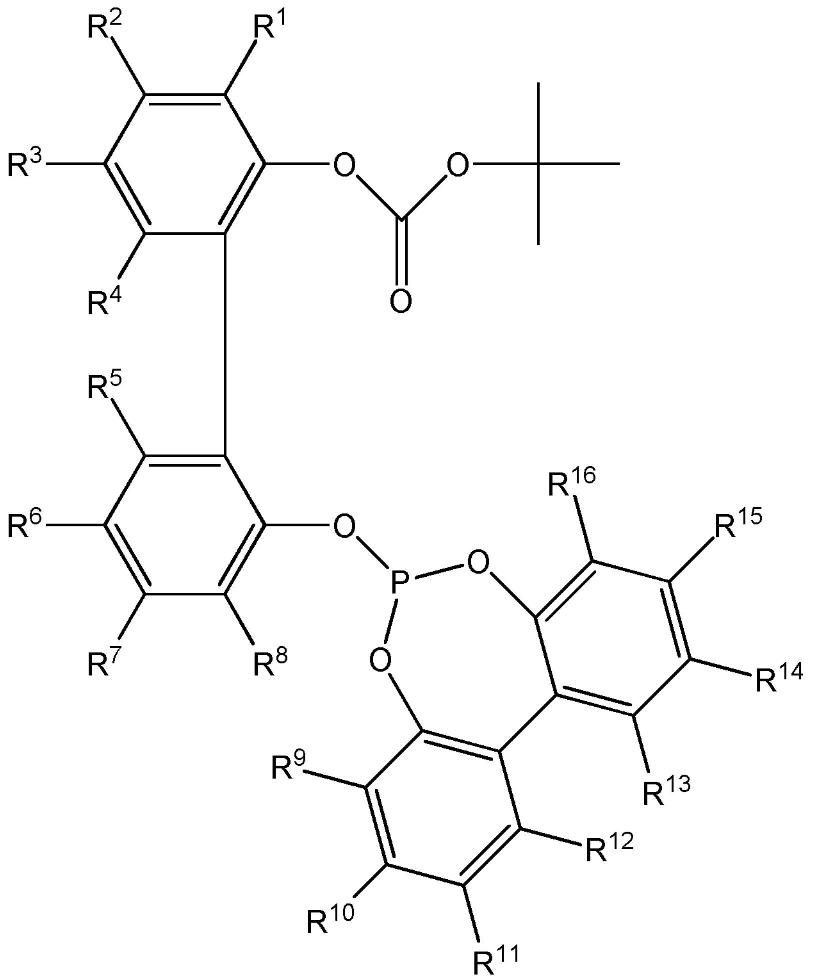

**III**

wobei $R^9$, $R^{10}$, $R^{11}$, $R^{12}$, $R^{13}$, $R^{14}$, $R^{15}$, $R^{16}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I), -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3H$, -$SO_3Na$, -$NO_2$, -CN, -$NH_2$, -N[($C_1$-$C_{12}$)-Alkyl]$_2$.

In einer Ausführungsform sind $R^9$, $R^{10}$, $R^{11}$, $R^{12}$, $R^{13}$, $R^{14}$, $R^{15}$, $R^{16}$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl, - CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl,-COOH, -OH, -$NH_2$, -N[($C_1$-$C_{12}$)-Alkyl]$_2$.

In einer Ausführungsform sind $R^9$, $R^{10}$, $R^{11}$, $R^{12}$, $R^{13}$, $R^{14}$, $R^{15}$, $R^{16}$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl,-N[($C_1$-$C_{12}$)-Alkyl]$_2$.

In einer Ausführungsform sind $R^9$, $R^{10}$, $R^{11}$, $R^{12}$, $R^{13}$, $R^{14}$, $R^{15}$, $R^{16}$ jeweils unabhängig voneinander ausgewählt aus:

-H, -($C_1$-$C_{12}$)-Alkyl, -O-($C_1$-$C_{12}$)-Alkyl, -($C_6$-$C_{20}$)-Aryl

wobei Alkyl 1-12 Kohlenstoffatome, bevorzugt 1-10 Kohlenstoffatome z.B. primäre, sekundäre oder tertiäre Alkylgruppen, wie Methyl-, Ethyl-, n-Propyl-, Isopropyl-, Butyl-, sec-Butyl-, t-Butyl-, t-Butylethyl-, t-Butylpropyl-, n-Hexyl-, Amyl-, sec-Amyl-, t-Amyl-, iso-Octyl-, 2-Ethylhexyl-, Decyl-, Dodecyl- und Octadecylgruppen umfasst.

In einer Ausführungsform stehen $R^{11}$ und $R^{14}$ für -O-($C_1$-$C_{12}$)-Alkyl.

In einer Ausführungsform stehen $R^{11}$ und $R^{14}$ für -OMe.

In einer Ausführungsform stehen $R^9$ und $R^{16}$ für -($C_1$-$C_{12}$)-Alkyl.

In einer Ausführungsform stehen $R^9$ und $R^{16}$ für *tert*-Butyl.

In einer Ausführungsform stehen $R^9$, $R^{11}$, $R^{14}$ und $R^{16}$ für -($C_1$-$C_{12}$)-Alkyl.

In einer Ausführungsform stehen $R^9$, $R^{11}$, $R^{14}$ und $R^{16}$ für Methyl.

In einer Ausführungsform stehen $R^9$, $R^{11}$, $R^{14}$ und $R^{16}$ für *tert*-Butyl.

In einer Ausführungsform stehen sind $R^9$, $R^{10}$, $R^{11}$, $R^{12}$, $R^{13}$, $R^{14}$, $R^{15}$ und $R^{16}$ für -H.

In einer Ausführungsform weist der Ligand die allgemeine Strukturen **IV** auf:

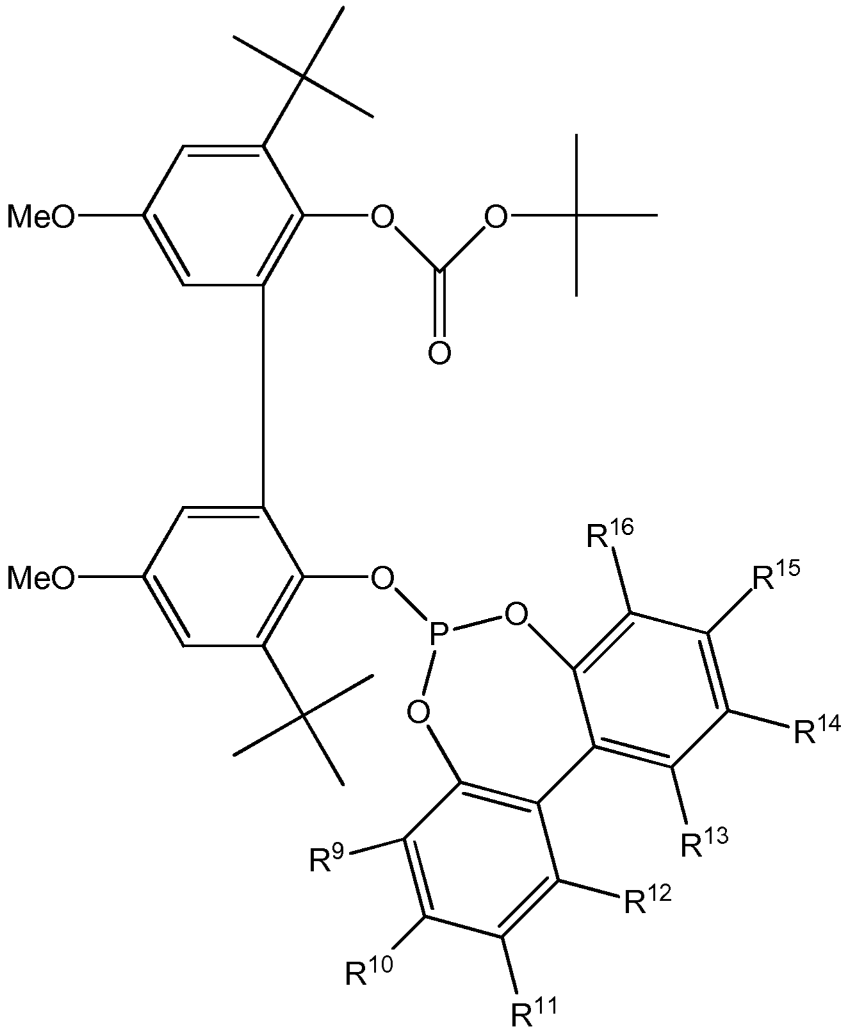

IV

wobei $R^9$, $R^{10}$, $R^{11}$, $R^{12}$, $R^{13}$, $R^{14}$, $R^{15}$, $R^{16}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen (wie Cl, F, Br, I), -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)Alkyl, -CO-($C_6$-$C_{20}$)-Aryl, -COOH, -OH, -$SO_3H$, -$SO_3Na$, -$NO_2$, -CN, -$NH_2$, -N[($C_1$-$C_{12}$)-Alkyl]$_2$.

In einer Ausführungsform sind $R^9$, $R^{10}$, $R^{11}$, $R^{12}$, $R^{13}$, $R^{14}$, $R^{15}$, $R^{16}$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl, - CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl,-COOH, -OH, -$NH_2$; -N[($C_1$-$C_{12}$)-Alkyl]$_2$.

In einer Ausführungsform sind $R^9$, $R^{10}$, $R^{11}$, $R^{12}$, $R^{13}$, $R^{14}$, $R^{15}$, $R^{16}$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl, - N[($C_1$-$C_{12}$)-Alkyl]$_2$.

In einer Ausführungsform sind $R^9$, $R^{10}$, $R^{11}$, $R^{12}$, $R^{13}$, $R^{14}$, $R^{15}$, $R^{16}$ jeweils unabhängig voneinander ausgewählt aus:

-H, -($C_1$-$C_{12}$)-Alkyl, -O-($C_1$-$C_{12}$)-Alkyl, -($C_6$-$C_{20}$)-Aryl;

wobei Alkyl 1-18 Kohlenstoffatome, bevorzugt 1-10 Kohlenstoffatome z.B. primäre, sekundäre oder tertiäre Alkylgruppen,

wie Methyl-, Ethyl-, n-Propyl-, Isopropyl-, Butyl-, sec-Butyl-, t-Butyl-, t-Butylethyl-, t-Butylpropyl-, n-Hexyl-, Amyl-, sec-Amyl-, t-Amyl-, iso-Octyl-, 2-Ethylhexyl-, Decyl-, Dodecyl- und Octadecylgruppen umfasst.

In einer Ausführungsform stehen $R^{11}$ und $R^{14}$ für -O-($C_1$-$C_{12}$)-Alkyl.

In einer Ausführungsform stehen $R^{11}$ und $R^{14}$ für -OMe.

In einer Ausführungsform stehen $R^9$ und $R^{16}$ für -($C_1$-$C_{12}$)-Alkyl.

In einer Ausführungsform stehen $R^9$ und $R^{16}$ für *tert*-Butyl.

In einer Ausführungsform stehen $R^9$, $R^{11}$, $R^{14}$ und $R^{16}$ für -($C_1$-$C_{12}$)-Alkyl.

In einer Ausführungsform stehen $R^9$, $R^{11}$, $R^{14}$ und $R^{16}$ für Methyl.

In einer Ausführungsform stehen $R^9$, $R^{11}$, $R^{14}$ und $R^{16}$ für *tert*-Butyl.

In einer Ausführungsform stehen sind $R^9$, $R^{10}$, $R^{11}$, $R^{12}$, $R^{13}$, $R^{14}$, $R^{15}$ und $R^{16}$ für -H.

Neben den Liganden wird auch ein Komplex beansprucht, welcher diese Liganden umfasst.

Komplex umfassend:

- einen zuvor beschriebenen Liganden,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung des Liganden als Ligand in einem Ligand-Metall-Komplex zur Katalyse einer Hydroformylierungsreaktion beansprucht.

Verwendung eines zuvor beschriebenen Liganden in einem Ligand-Metall-Komplex zur Katalyse einer Hydroformylierungsreaktion.

Das Verfahren bei dem der Ligand als Ligand in einem Ligand-Metall-Komplex zur Umsetzung eines Olefins zu einem Aldehyd eingesetzt wird, wird ebenfalls beansprucht.

Verfahren umfassend die Verfahrensschritte:

a) Vorlegen eines Olefins,
b) Zugabe eines zuvor beschriebenen Komplexes,
oder eines zuvor beschriebenen Liganden und einer Verbindung, welche ein Metallatom ausgewählt aus: Rh, Ru, Co, Ir aufweist,
c) Zuführen von $H_2$ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

Die Reaktion wird bei üblichen Bedingungen durchgeführt.

Bevorzugt sind eine Temperatur von 80 °C bis 160 °C und ein Druck von 1 bis 300 bar.

Besonders bevorzugt sind eine Temperatur von 100 °C bis 160 °C und ein Druck von 15 bis 250 bar.

In einer bevorzugten Ausführungsform ist das Metall Rh.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende $C_6$-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende $C_8$-Olefingemisch (Di-n-buten, Di-iso-buten), Nonene, 2-oder 3-Methyloctene, das bei der Trimerisierung von Propen anfallende $C_9$-Olefingemisch (Tripropen), Decene, 2-Ethyl-1-octen, Dodecene, das bei der Tetramerisierung von Propen oder der Trimerisierung von Butenen anfallende $C_{12}$-Olefingemisch (Tetrapropen oder Tributen), Tetradecene, Hexadecene, das bei der Tetramerisierung von Butenen anfallende $C_{16}$-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden $\alpha$-Olefine, endständig verzweigte, innenständige und innenständig verzweigte Olefine hydroformyliert werden. Bemerkenswert ist dabei die hohe Ausbeute an endständig hydrofomyliertem Olefin, selbst wenn im Edukt nur ein geringer Anteil an Olefinen mit endständiger Doppelbindung vorhanden war.

Im Folgenden soll die Erfindung anhand von Ausführungsbeispielen näher erläutert werden.

Die folgenden siebzig Strukturen (**1** bis **70**) zeigen mögliche Ausführungsbeispiele des Liganden:

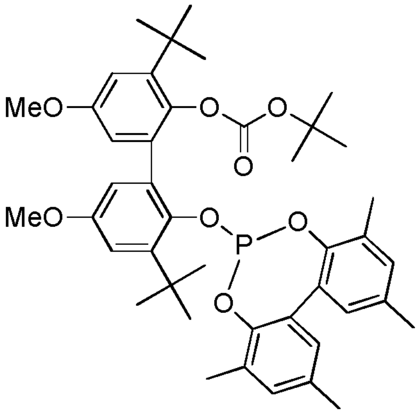

1

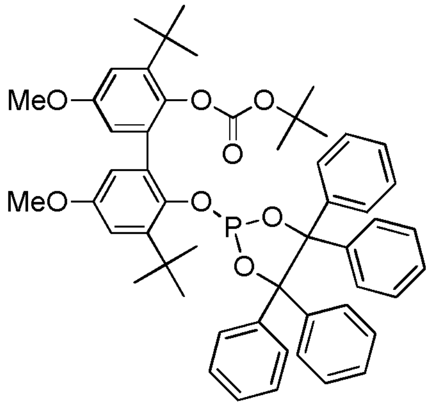

2

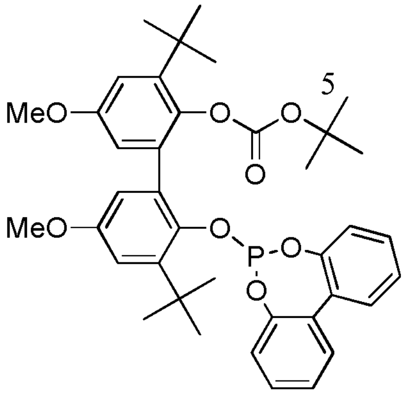

3

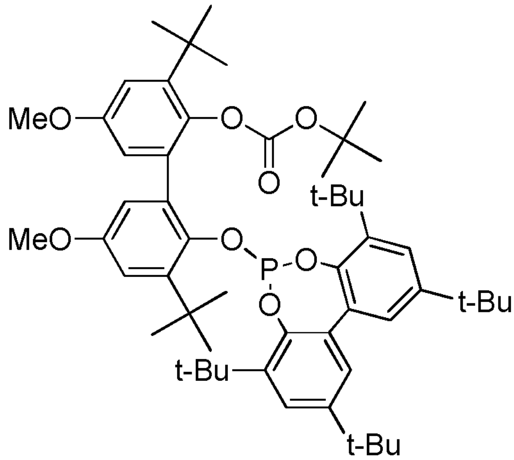

4

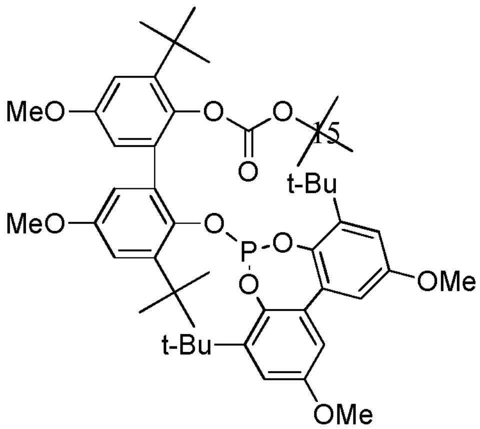

5

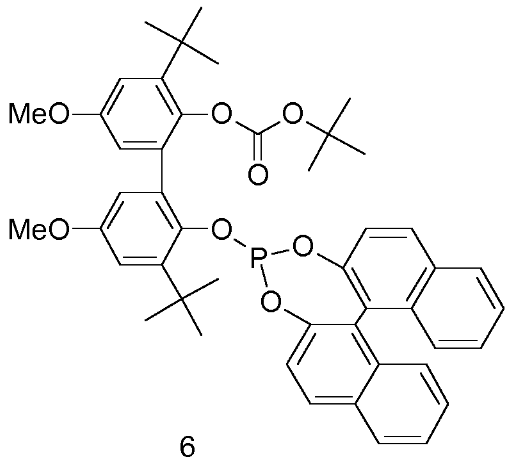

6

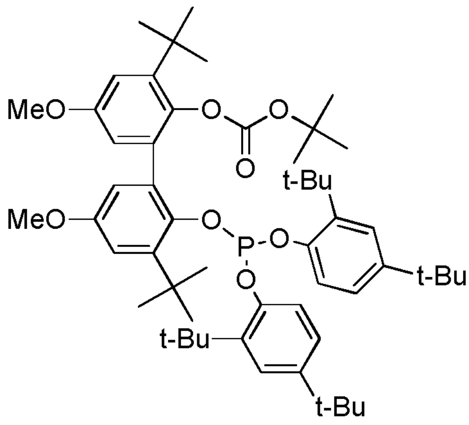

7

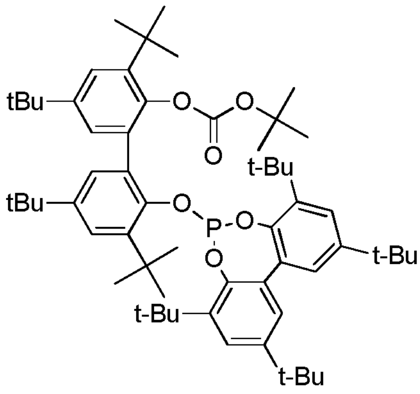

8

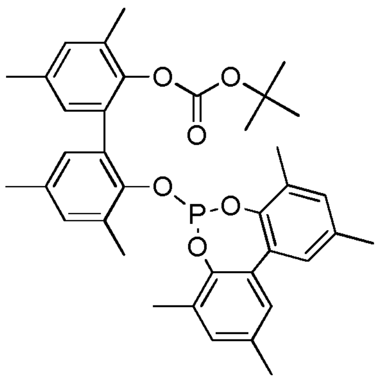

9

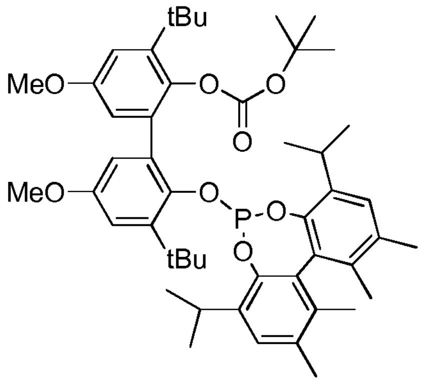

10

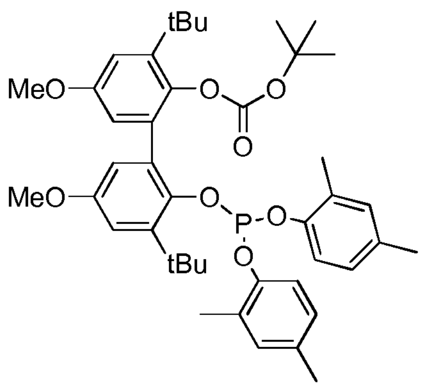

11

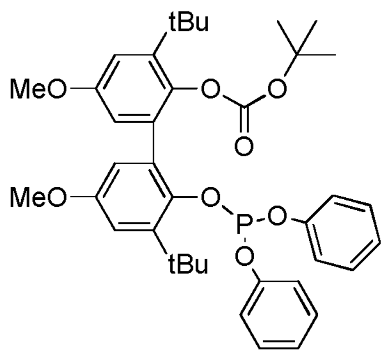

12

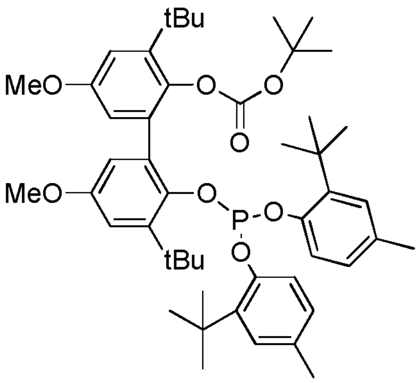

13

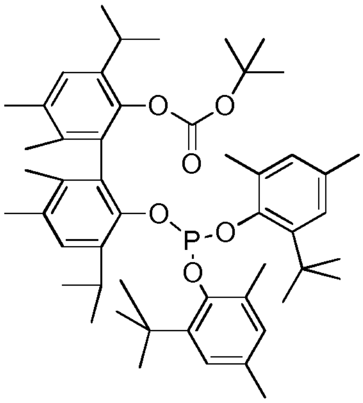

14

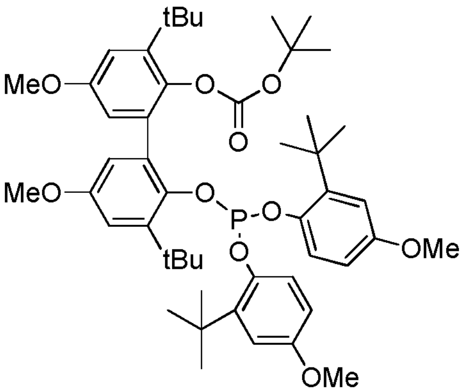

15

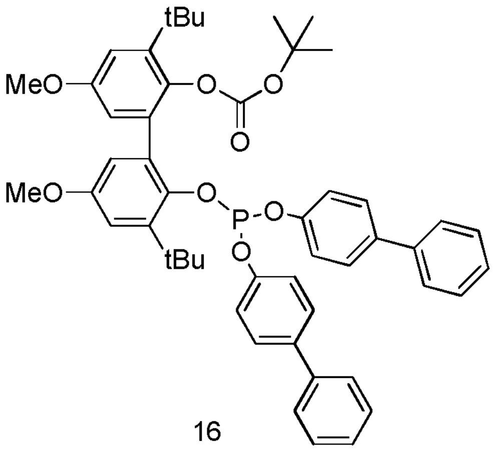

16

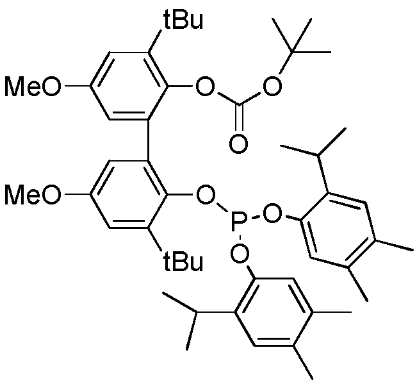

17

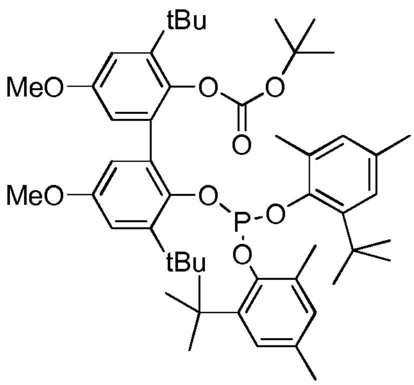

18

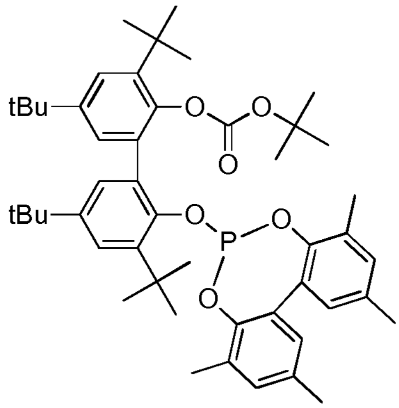

19

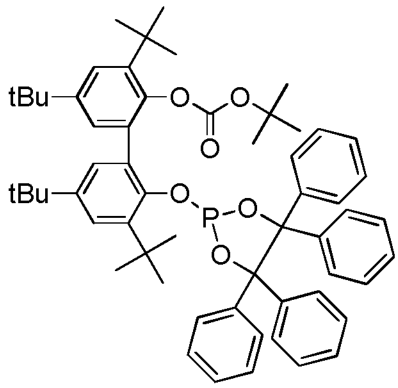

20

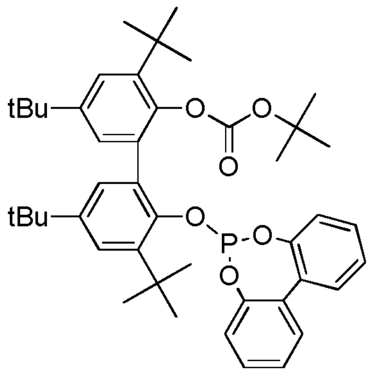

21

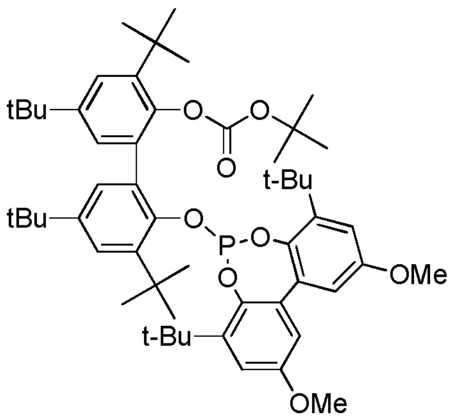

22

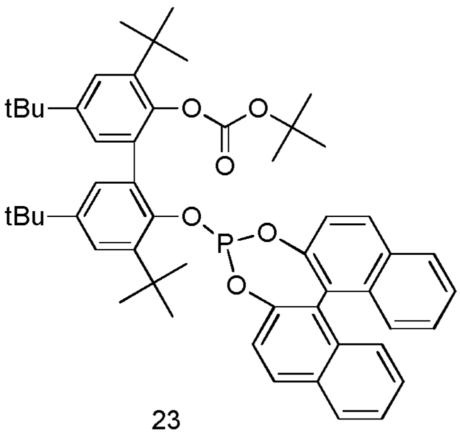

23

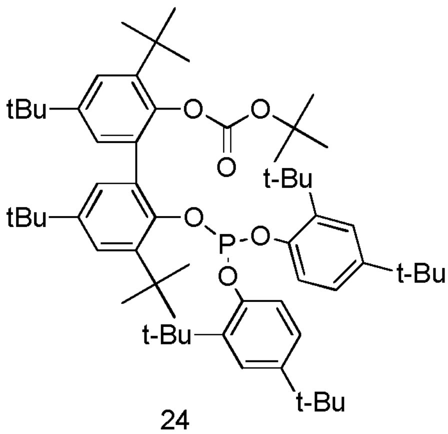

24

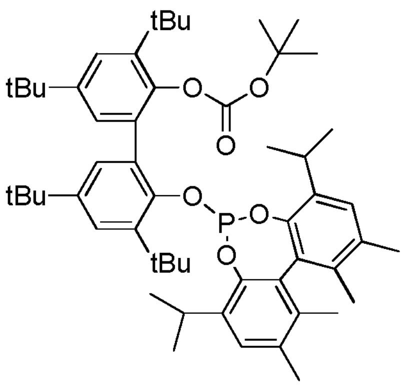

25

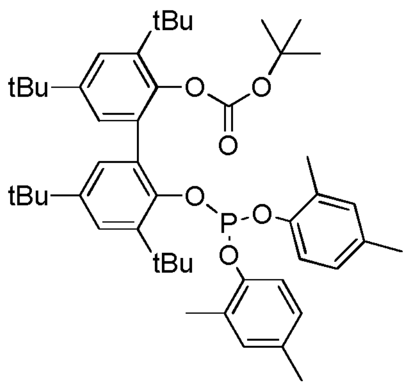

26

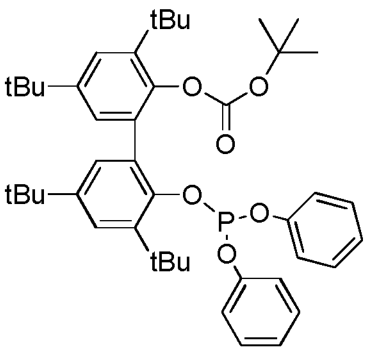

27

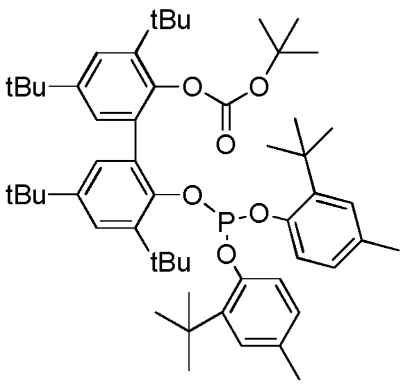

28

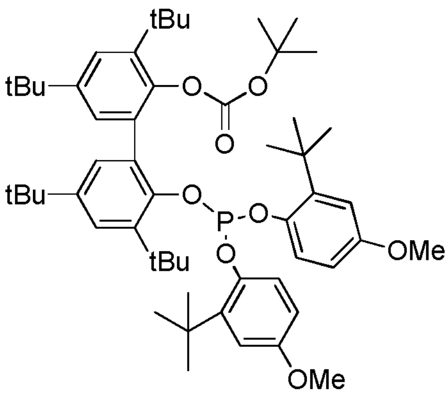

29

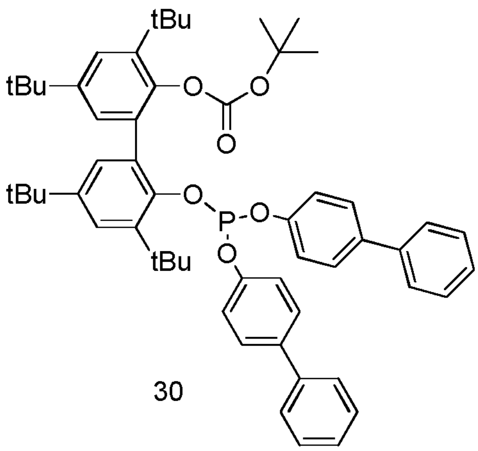

30

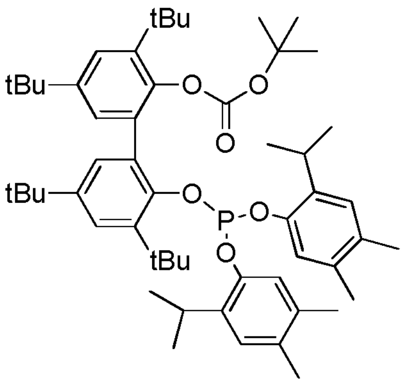

31

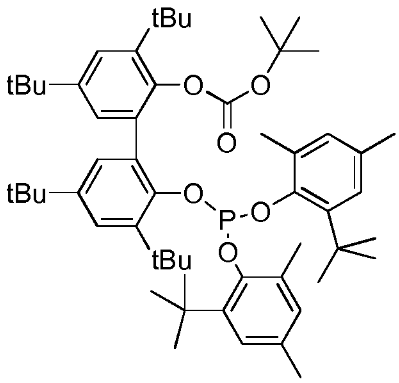

32

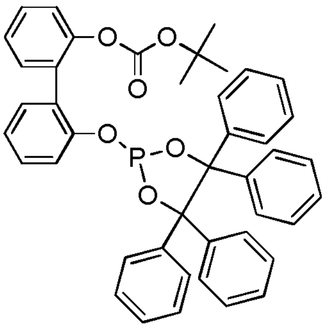

33

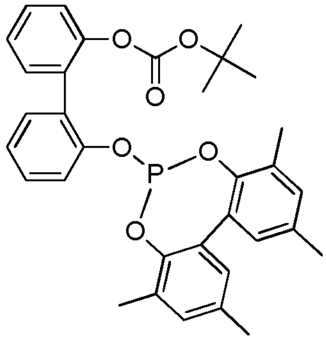

34

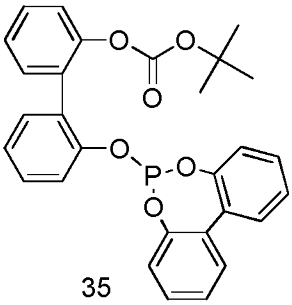

35

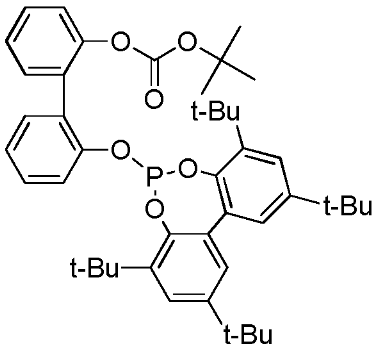

36

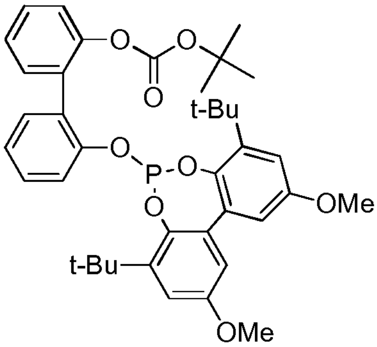

37

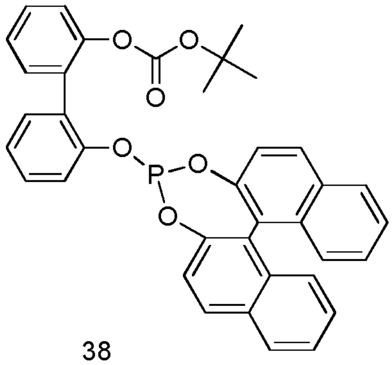

38

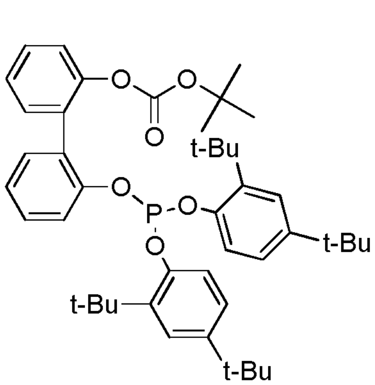

39

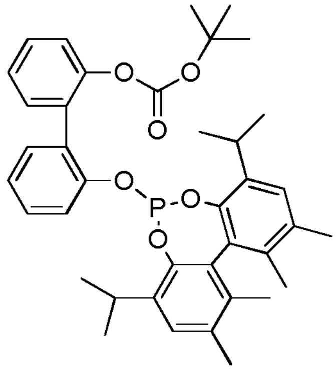

40

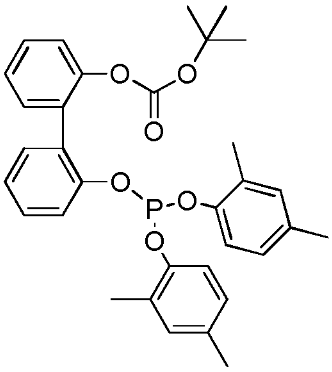

41

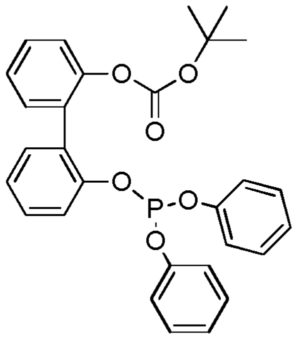

42

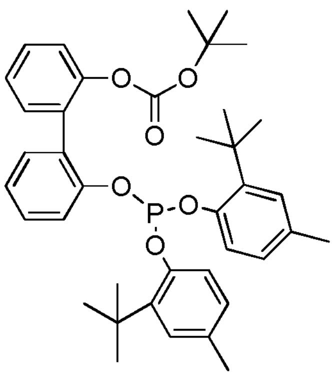

43

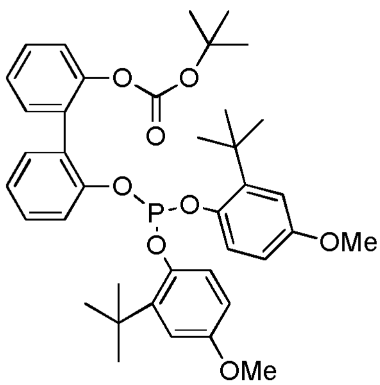

44

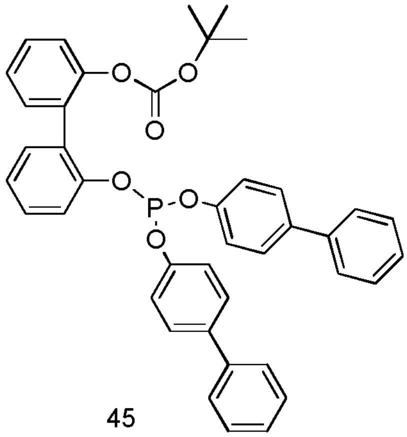

45

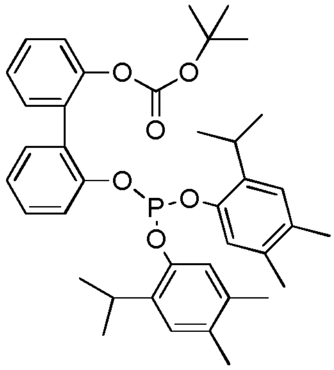

46

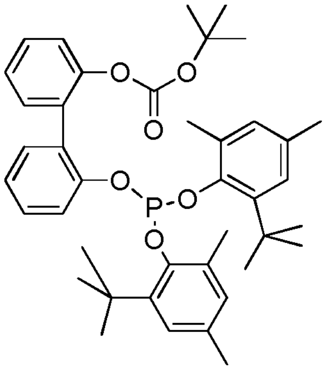

47

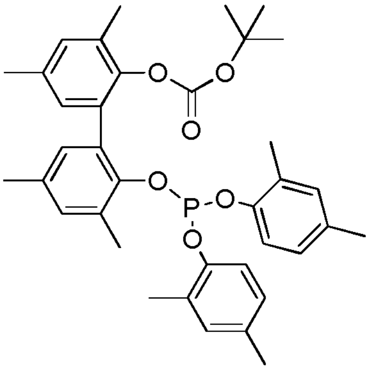

48

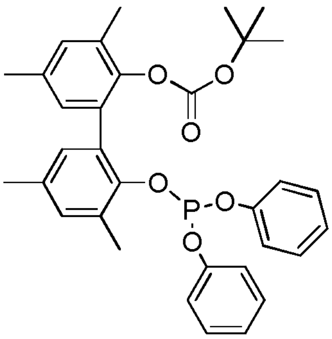

49

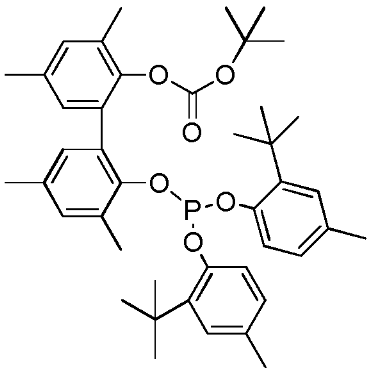

50

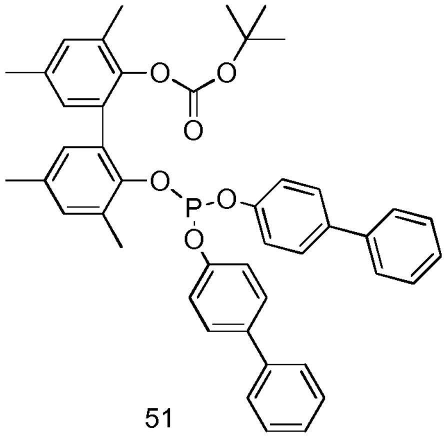

51

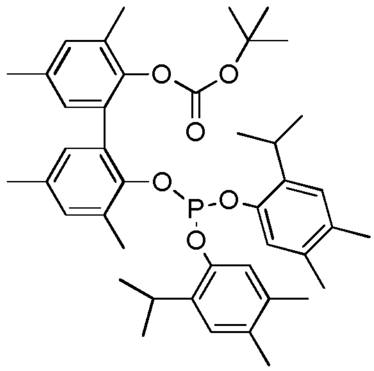

52

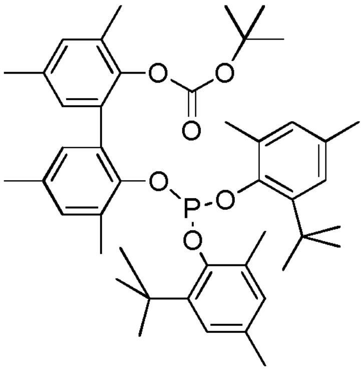

53

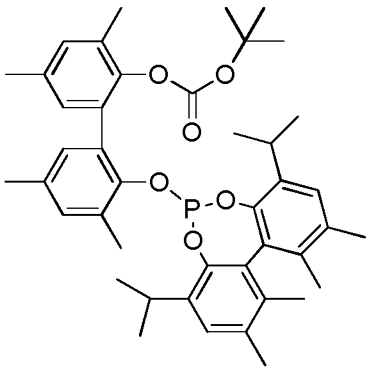

54

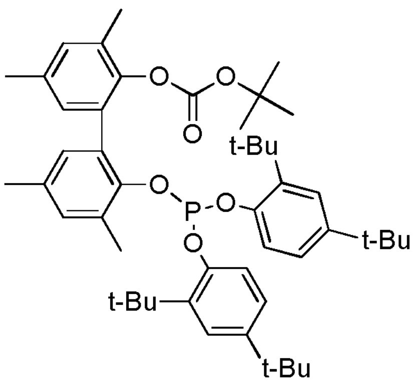

55

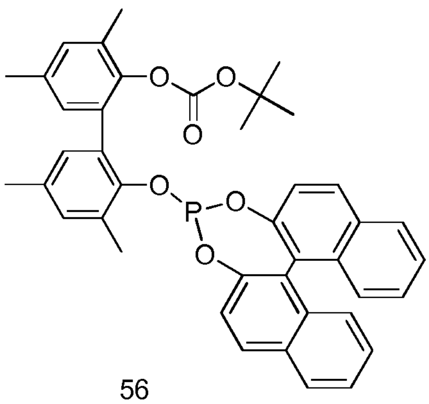

56

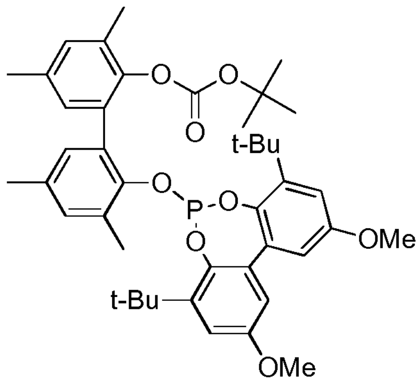

57

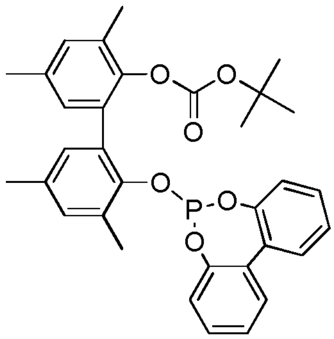

58

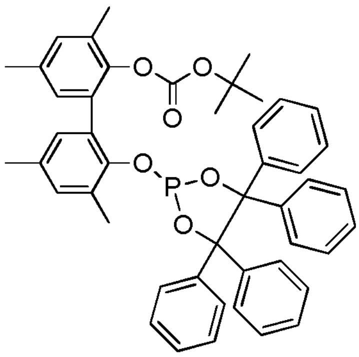

59

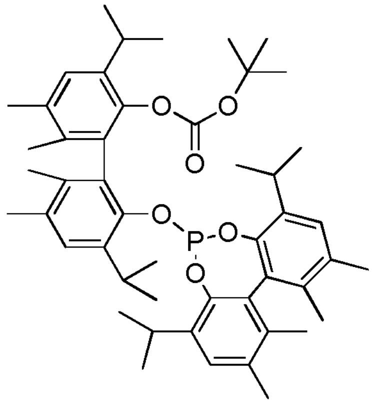

60

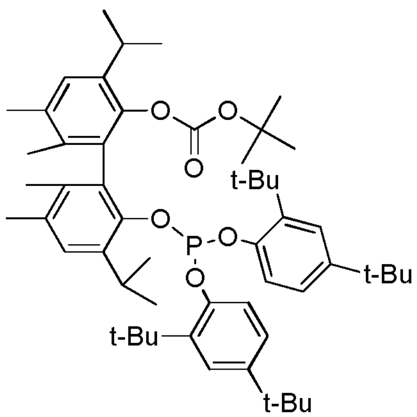

61

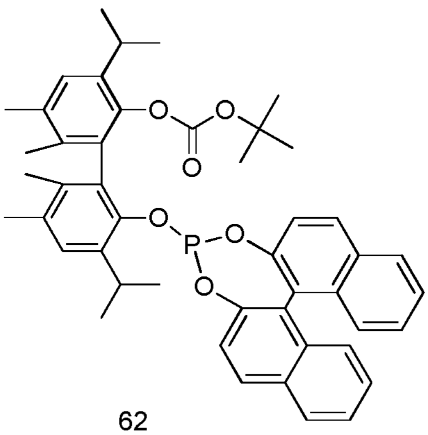

62

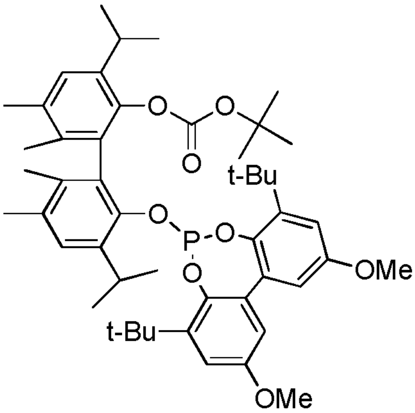

63

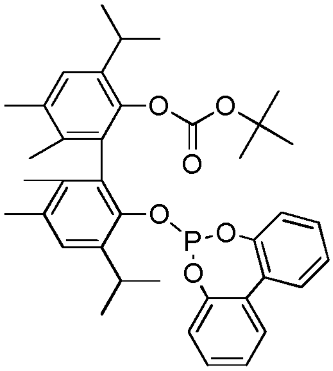

64

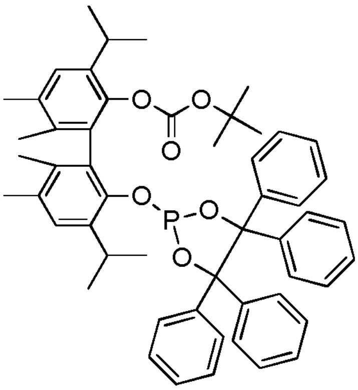

65

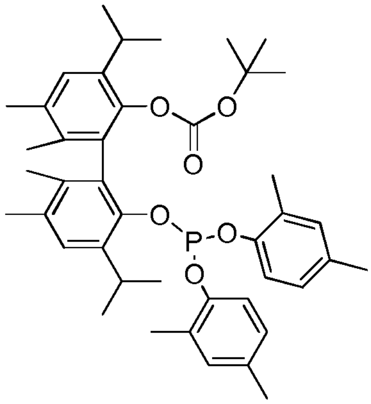

66

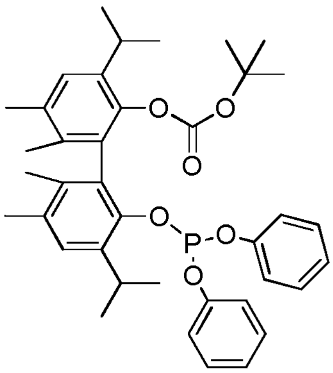

67

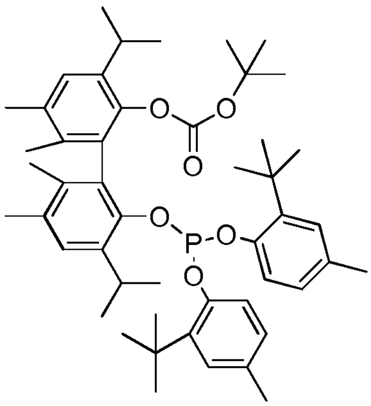

68

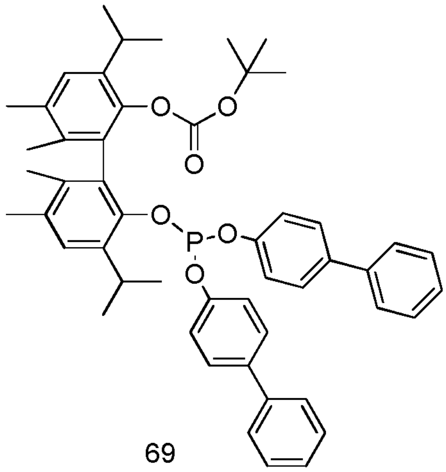

69

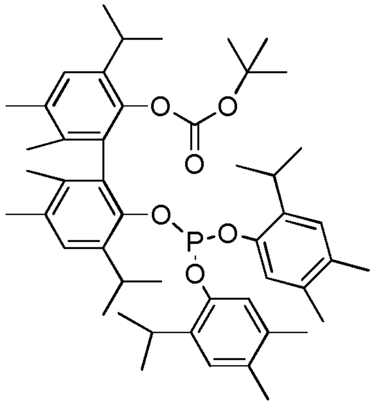

70

## Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego, Christina Chai, Butterworth Heinemann (Elsevier), 6. Auflage, Oxford 2009).

Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der $^{31}P$-NMR-Signale erfolgte gemäß: $SR_{31P} = SR_{1H} * (BF_{31P} / BF_{1H}) = SR_{1H} * 0{,}4048$. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte mittels eines Bruker Avance 300 bzw. Bruker Avance 400, die gaschromatografische Analyse an Agilent GC 7890A.

Die erfindungsgemäßen Liganden lassen sich hierbei auf verschiedenen Wegen herstellen. Drei mögliche Wege sind in den nachfolgenden Schemata (A bis C) dargestellt.

Die dargestellten Reaktionswege sind nur exemplarisch und in einer stark vereinfachten Form dargestellt. So kann nach Bedarf in allen Schritten zusätzlich Base verwendet werden. Weiterhin können die in den einzelnen Synthesestufen genannten Basen durch andere, dem Fachmann bekannte und kommerziell verfügbare Basen, substituiert werden.

Reaktionsweg A:

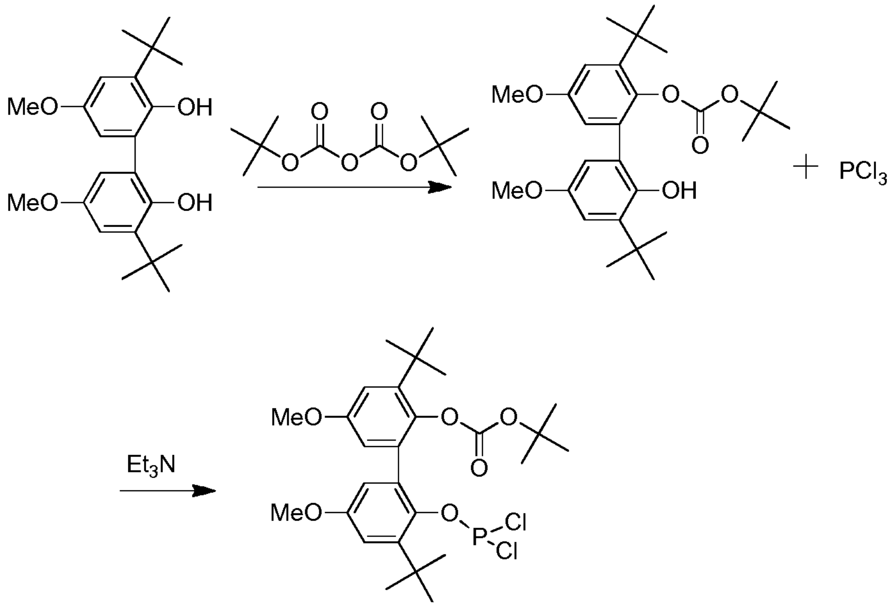


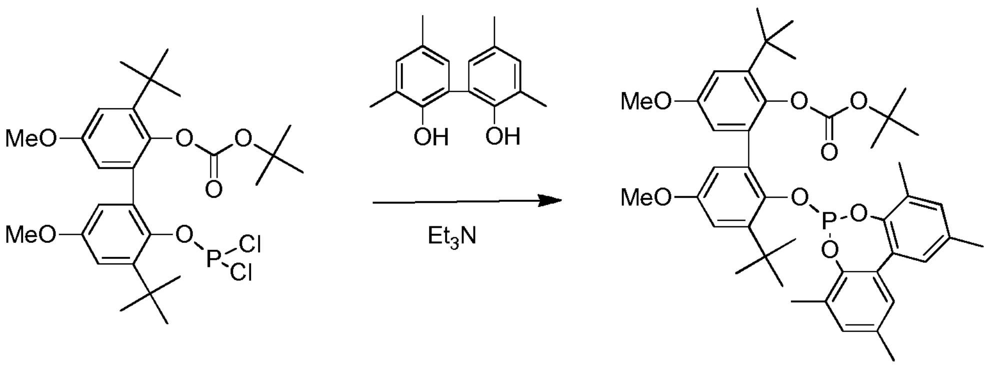


**1**

Reaktionsweg B:

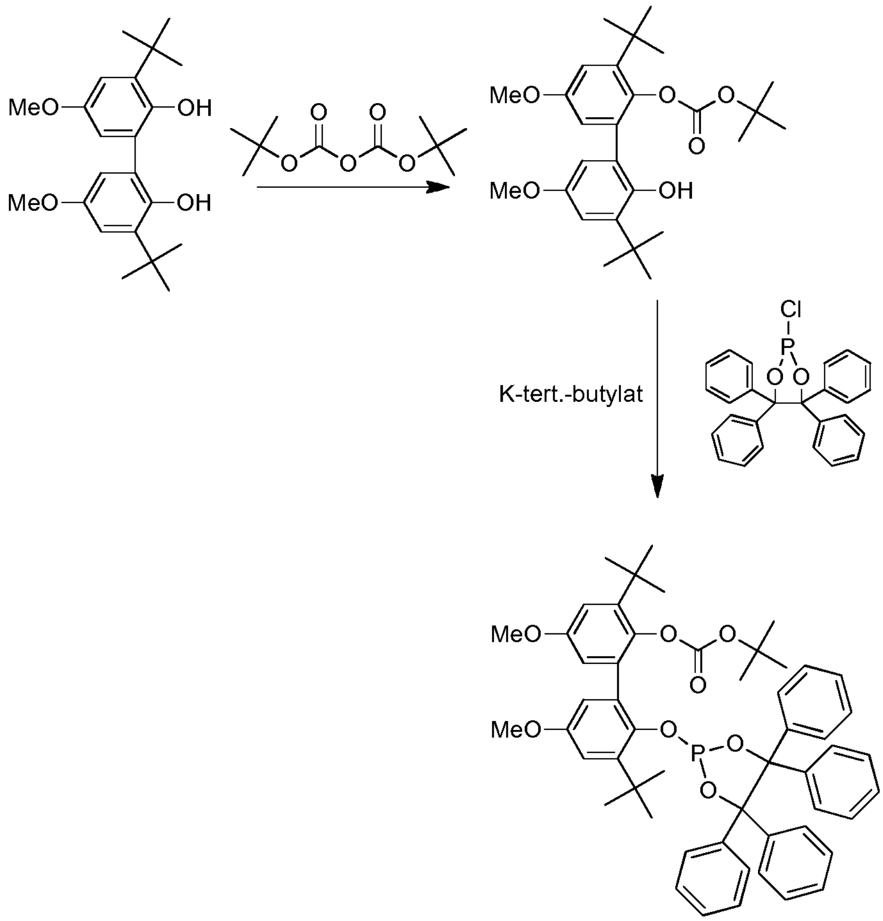


**2**

Reaktionsweg C:

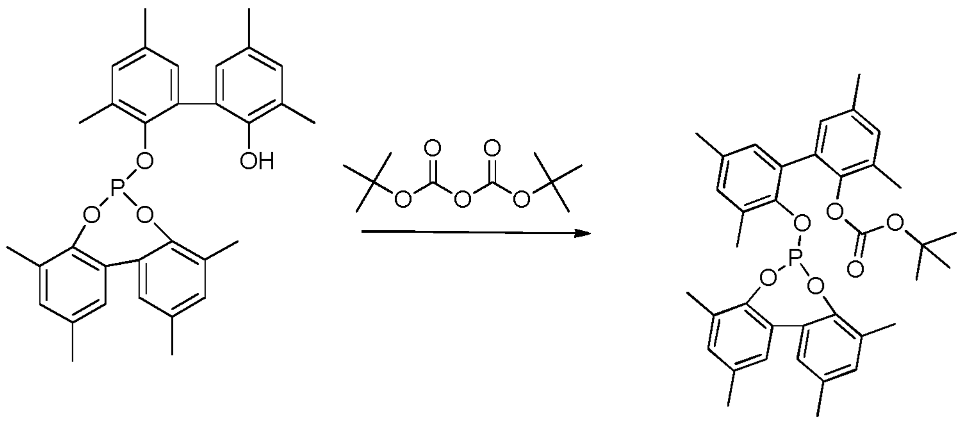

**9**

<u>**Synthese von Ligand 1**</u>

<u>Reaktionsschema:</u>

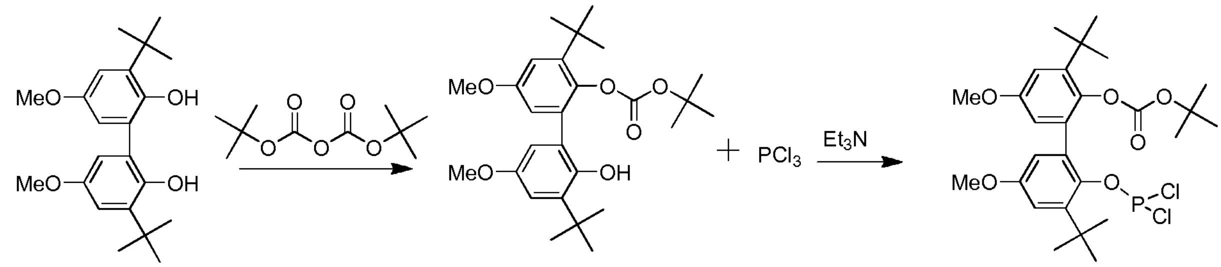


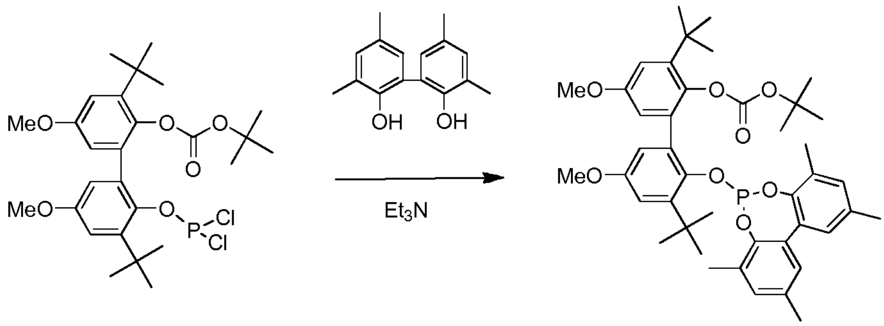


Einführung der BOC-Gruppe:

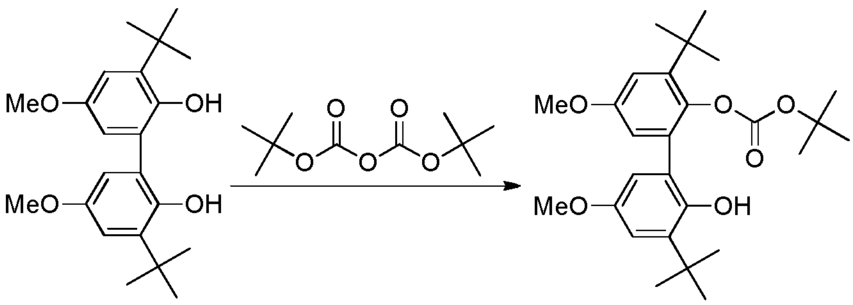

In einem 2L Schlenkkolben werden 400 mmol (143,8 g) des 3,3'-Di-tert.-butyl-5,5'-dimethoxy-[1,1'-biphenyl]-2,2'diol und 40 mmol (4,8 g) N,N-Dimethylaminopyridin (DMAP) in 900 mL $CH_2Cl_2$ gelöst. Anschließend wurden bei Raumtemperatur 400 mmol (88 g) Di-tert-butyldicarbonat in 280 ml $CH_2Cl_2$ gelöst, in einen 500 ml Tropftrichter überführt und innerhalb einer Stunde bei 32 °C zu der Biphenol/DMAP Lösung getropft. Die Lösung wurde über Nacht bei Raumtemperatur gerührt. Am nächsten Morgen wird das Lösungsmittel unter vermindertem Druck entfernt. Der leicht wachsartige, rötliche Rückstand wurde mit 800 ml n-Heptan versetzt und über Nacht gerührt. Dabei erhielt man einen weißen Rückstand, der abfiltriert, zweimal mit 50 ml n-Heptan nachgewaschen und dann getrocknet wurde. Das Zielprodukt konnte als weißer Feststoff (161,6 g, 84%) erhalten werden. $^1$H-NMR (Tolul-$d_8$): 95% und weitere Verunreinigungen.

Umsetzung von tert-Butyl-(3,3'-di-tert-butyl-2'-hydroxy-5,5'-dimethoxy-[1,1'-biphenyl]-2-yl)-carbonat mit Phosphortrichlorid:

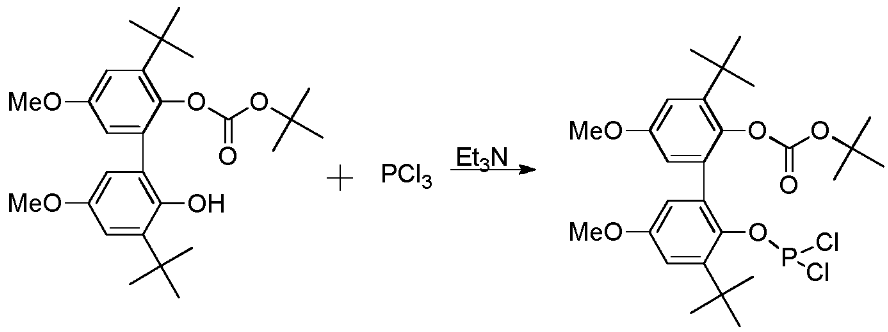


In einem sekurierten 250 ml Schlenkkolben wurden 12 g (0,026 mol) tert-Butyl-(3,3'-di-tert-butyl-2'-hydroxy-5,5'-dimethoxy-[1,1'-biphenyl]-2-yl)-carbonat unter Rühren in 120 ml getrocknetem Toluol und 12,8 ml (0,091 mol) Triethylamin gelöst.

In einem zweiten 500 ml Schlenkkolben wurden zunächst 100 ml getrocknetes Toluol mit 8,1 ml (0,091 mol) Phosphortrichlorid verrührt. Anschließend wurde die Phosphortrichlorid-Toluol-Lösung zu der zuvor hergestellten Carbonat-Amin-Toluol-Lösung innerhalb von 30 Minuten bei Raumtemperatur zugetropft. Nach vollständiger Zugabe wurde für 30 Minuten auf 80 °C erwärmt und über Nacht auf Raumtemperatur abgekühlt.

Am nächsten Morgen wurde die Mischung filtriert, mit 50 ml getrocknetem Toluol nachgewaschen und das Filtrat bis zur Trockne eingeengt. Das Zielprodukt konnte als Feststoff (13,1 g, 89%) erhalten werden. $^{31}$P-NMR (202,4 MHz, Toluol-$d_8$): 203,2 und 203,3 ppm (100%).

<u>Umsetzung von tert-Butyl-(3,3'-di-tert-butyl-2'-((dichlorophosphino)oxy)-5,5'-dimethoxy-[1,1'-biphenyl]-2-yl)-carbonat mit 3,3',5,5'-Tetramethyl-(1,1'-biphenyl)-2,2'-diol</u>

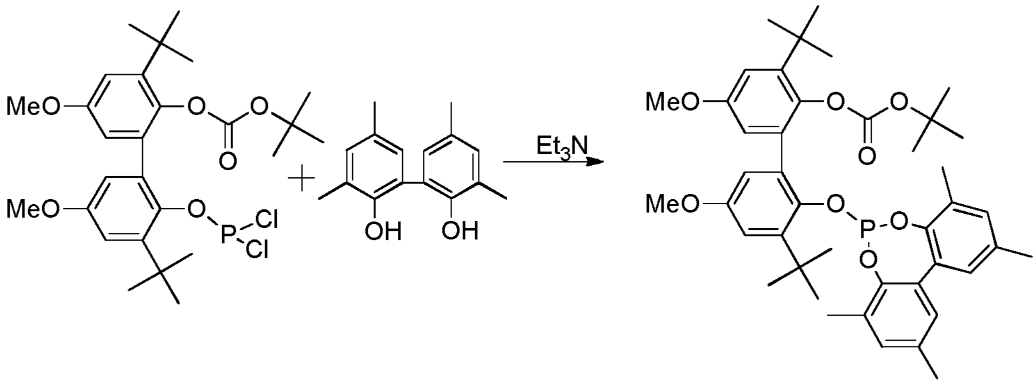


In einem sekurierten 1L Schlenkkolben wurden 24,7 g (0,044 mol) tert-Butyl-(3,3'-di-tert-butyl-2'-((dichlorophosphino)oxy)-5,5'-dimethoxy-[1,1'-biphenyl]-2-yl)-carbonat in 400 ml Acetonitril gelöst. In einem zweiten sekurierten Schlenkkolben (1 L) wurden 10,8 g (0,044 mol) 3,3',5,5'-Tetramethyl-(1,1'-biphenyl)-2,2'-diol in 200 ml Acetonitril und 13,1 ml (0,011 mol) getrocknetem Triethylamin unter Rühren gelöst. Anschließend wurde zu der Biphenol-Triethylamin-Lösung langsam die Chlorophosphit-Lösung hinzugetropft und über Nacht gerührt.

Der Ansatz wurde daraufhin filtriert und der Rückstand zweimal mit 15 ml Acetonitril gewaschen. Das Filtrat wurde unter vermindertem Druck eingeengt bis ein Niederschlag ausfiel. Dieser wurde filtriert und getrocknet. Das Zielprodukt konnte als weißer Feststoff (28,5 g, 87%) erhalten werden. $^{31}$P-NMR (202,4 MHz,Toluol-$d_8$):139,4 ppm (98,5%).

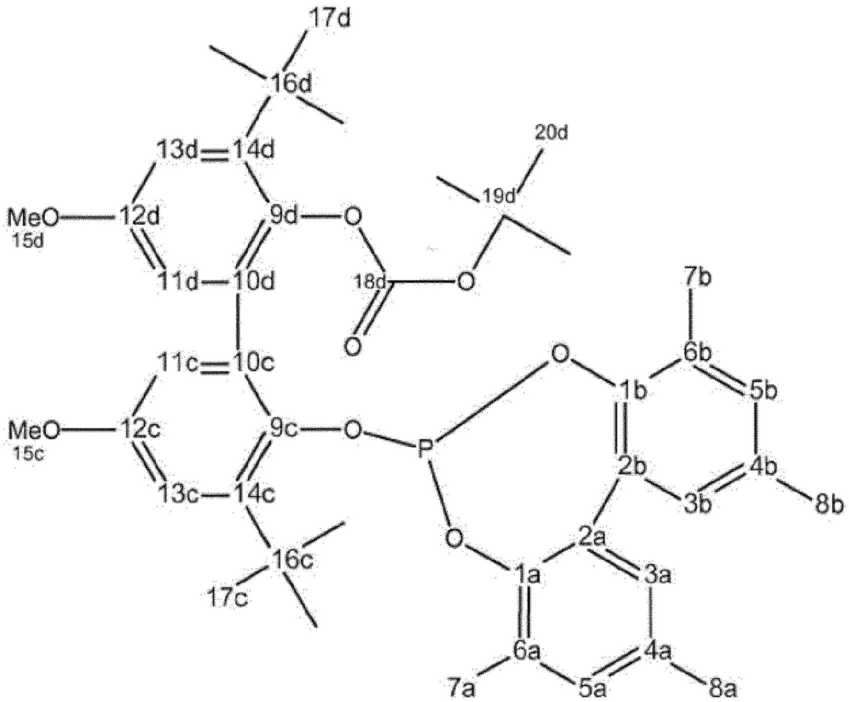

(**1**)

Spektrometer: Bruker Avance 500 MHz FT-Spektrometer Lösungsmittel: 1,1,2,2-Tetrachlorethan (TCE) Temperatur: 353K (80°C)
Referenzierung: $^{1}$H-NMR, $^{13}$C-NMR: TMS = 0

$$^{31}P\text{-NMR: } SR_{31P} = SR_{1H} * (BF_{31P}/BF_{1H}) = SR_{1H} * 0{,}404807$$

Tabelle 1: Zuordnung $^{1}$H chemische Verschiebungen von 1

| δ / ppm | Intensität | Multiplizität | Zuordnung |
|---|---|---|---|
| 1,13 | 9H | s | 17d (tert-Butyl) |
| 1,20 | 9H | s | 20d (O-tert-Butyl) |
| 1,35 | 9H | s | 17c (tert-Butyl) |
| 2,02 | 3H | breites-s | 7b (Methyl) |
| 2,28 | 3H | s | 8b (Methyl) |
| 2,31/2,32 | 6H | breites-s | 7a (Methyl) / 8a (Methyl) |
| 3,75 | 3H | s | 15c (O-Methyl) |
| 3,78 | 3H | s | 15d (O-Methyl) |
| 6,68 | 1H | d | 11d |
| 6,83 | 1H | d | 11c |
| 6,88 | 2H (1+1) | m | 5b, 13d |
| 6,94 | 1H | d | 13c |
| 6,96 | 1H | s | 5a |

(fortgesetzt)

| δ / ppm | Intensität | Multiplizität | Zuordnung |
|---|---|---|---|
| 6,99 | 2H (1+1) | s | 3a + 3b |
| s: Singulett<br>d: Duplett<br>t: Triplett<br>q: Quartett<br>m: Multiplett | | | |

Tabelle 2: Zuordnung $^{13}C$ chemische Verschiebungen von 1

| δ / ppm | Intensität | Gruppe | Zuordnung |
|---|---|---|---|
| 16,35 | 1 | $CH_3$ | 7b |
| 16,61 | 1 | $CH_3$ | 7a |
| 20,66 | 1 | $CH_3$ | 8b |
| 20,71 | 1 | $CH_3$ | 8a |
| 27,38 | 3 | $CH_3$ | 20d |
| 30,27 | 3 | $CH_3$ | 17d |
| 30,41 | 3 | $CH_3$ | 17c |
| 34,85 | 1 | $C_q$ | 16c |
| 35,02 | 1 | $C_q$ | 16d |
| 55,67 | 1 | $CH_3$ | 15d |
| 55,79 | 1 | $CH_3$ | 15c |
| 81,67 | 1 | $C_q$ | 19d |
| 113,86 | 1 | CH | 11c |
| 114,01 | 1 | CH | 11d |
| 114,14 | 1 | CH | 13c |
| 114,60 | 1 | CH | 13d |
| 127,65 | 1 | CH | 3b |
| 127,88 | 1 | CH | 3a |
| 128,72 | 1 | $C_q$ | 10d |
| 130,01 | 1 | $C_q$ | 6b |
| 130,77 | 1 | $C_q$ | 6a |
| 130,86 | 1 | CH | 5b |
| 130,92 | 1 | CH | 5a |
| 131,37 | 1 | $C_q$ | 2b |
| 131,50 | 1 | $C_q$ | 2a |
| 133,49 | 1 | $C_q$ | 4b |
| 133,65 | 1 | $C_q$ | 4a |
| 135,68 | 1 | $C_q$ | 10c |
| 142,09 | 1 | $C_q$ | 9d |
| 142,37 | 1 | $C_q$ | 14d |

(fortgesetzt)

| δ / ppm | Intensität | Gruppe | Zuordnung |
|---|---|---|---|
| 143,60 | 1 | $C_q$ | 9c |
| 144,05 | 1 | $C_q$ | 14c |
| 145,88 | 1 | $C_q$ | 1b |
| 146,00 | 1 | $C_q$ | 1a |
| 151,27 | 1 | $C_q$ | 18d |
| 154,18 | 1 | $C_q$ | 12d |
| 157,19 | 1 | $C_q$ | 12c |

**Synthese von Ligand 2**

Umsetzung von tert-Butyl-(3,3'-di-tert-butyl-2'-hydroxy-5,5'-dimethoxy-[1,1'-biphenyl]-2-yl)-carbonat mit 2-Chloro-4,4,5,5-tetraphenyl-1,3,2-dioxaphospholan

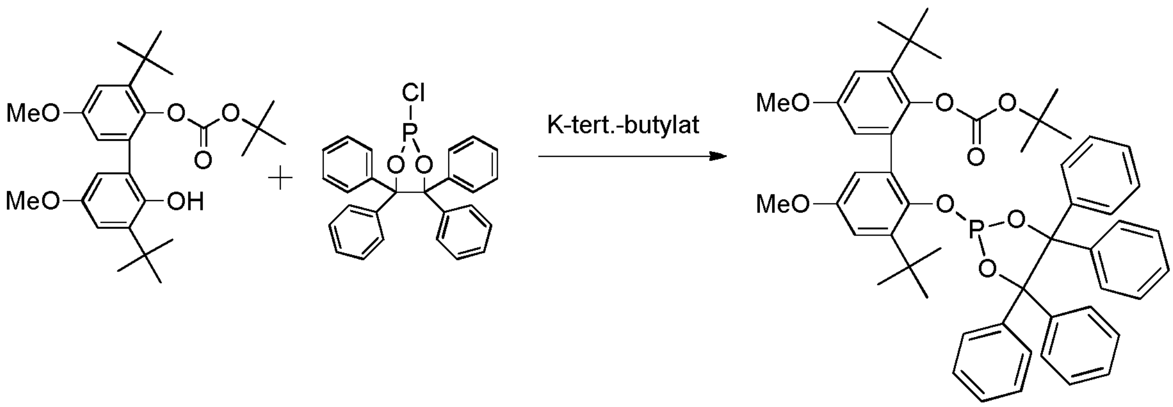


In einem sekurierten 250 ml Schlenkkolben wurden 9,1 g (0,021 mol) 2-Chloro-4,4,5,5-tetraphenyl-1,3,2-dioxaphospholan in 75 ml getrocknetem Toluol aufgelöst.

In einem zweiten Schlenkkolben (250 ml) wurden 9,2 g (0,02 mol) tert-Butyl-(3,3'-di-tert-butyl-2'-hydroxy-5,5'-dimethoxy-[1,1'-biphenyl]-2-yl)-carbonat und 2,3 g (0,02 mol) Kalium-tert.-butylat unter Rühren in 75 ml getrocknetem Toluol gelöst.

Anschließend wurde zu der Chlorophosphit-Lösung langsam bei Raumtemperatur die Carbonat-Kalium-tert.-butylat-Toluol-Mischung getropft und über Nacht bei Raumtemperatur gerührt.

Im Anschluss wurde das Lösungsmittel unter vermindertem Druck entfernt. Der erhaltene Rückstand wurde 5 Stunden in 75 ml getrocknetem Acetonitril gerührt. Der Feststoff wurde filtriert, mit getrocknetem Acetonitril nachgewaschen und getrocknet. Das Zielprodukt konnte als weißer Feststoff (15,3 g, 90%) erhalten werden. $^{31}P$-NMR (202,4 MHz, Toluol-$d_8$):147,0 ppm (99%).

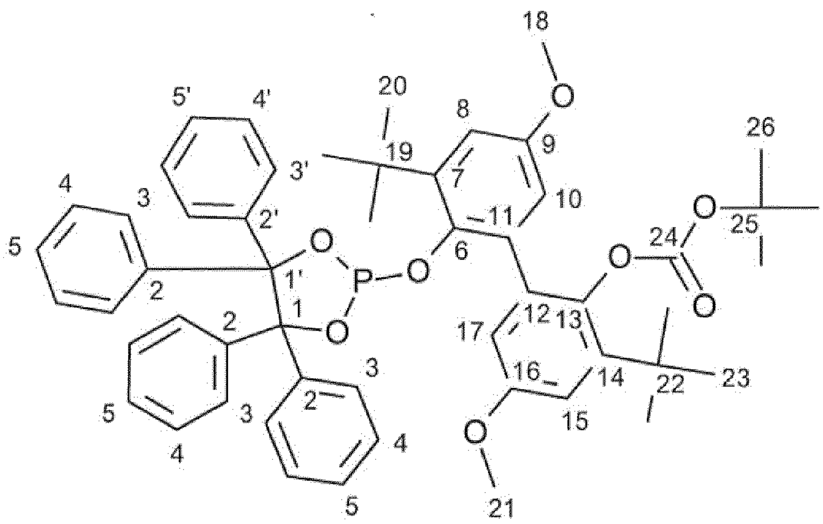

(**2**)

Referenzierung:

$$^{1}H\text{-NMR: TMS} = 0\text{ ppm},\ ^{31}P\text{-NMR: } SR_{31P} = SR_{1H} * (BF_{31P} / BF_{1H}) = SR_{1H} * 0{,}404807$$

Tabelle 3: Zuordnung der $^{13}C$ chemischen Verschiebungen von 2

| δ [ppm] | Intensität | Gruppe | Zuordnung |
|---|---|---|---|
| 157,31 | 1xC | $C_q$ | C9 |
| 155,29 | 1xC | $C_q$ | C16 |
| 152,22 | 1xC | $C_q$ | C24 |
| 143,68 | 1xC | $C_q$ | C13 |
| 143,66/143,54 | 2xC | $C_q$ | C7, C14 |
| 143,35 | 1xC | $C_q$ | C6 |
| 142,99/142,80 142,72/142,49 | 4xC | $C_q$ | C2 |
| 135,65 | 1xC | $C_q$ | C11 |
| 132,78 | 1xC | $C_q$ | C12 |
| 131,30/130,45 129,66/129,04 | 8xC | CH | C4 |
| 127,40-126,90 | 12 x C | CH | C3, C5 |
| 115,17 | 1xC | CH | C10 |
| 114,89 | 1xC | CH | C8 |
| 114,83 | 1xC | CH | C15 |
| 114,10 | 1xC | CH | C17 |
| 95,64/94,85 | 2xC | $C_q$ | C1, C1’ |

(fortgesetzt)

| δ [ppm] | Intensität | Gruppe | Zuordnung |
|---|---|---|---|
| 81,32 | 1xC | $C_q$ | C25 |
| 55,08 | 1xC | $CH_3$ | C21 |
| 55,04 | 1xC | $CH_3$ | C18 |
| 35,25 | 1xC | $C_q$ | C19 |
| 35,09 | 1xC | $C_q$ | C22 |
| 30,77 | 3xC | $CH_3$ | C20 |
| 30,64 | 3xC | $CH_3$ | C23 |
| 27,56 | 3xC | $CH_3$ | C26 |

Tabelle 4: Zuordnung der $^1$H chemischen Verschiebungen von 2

| δ [ppm] | Intensität | Multiplizität | Zuordnung |
|---|---|---|---|
| 7,54 | 4H | m | H3,H4,H5 H8, H10 H15, H17 |
| 7,21 | 3H | m | |
| 7,09 | 5H | m | |
| 6,99-6,89 | 6H | m | |
| 6,83 | 6H | m | |
| 3,50 | 3H | s | H19 |
| 3,41 | 3H | s | H22 |
| 1,39 | 9H | s | H20 |
| 1,31 | 9H | s | H23 |
| 1,21 | 9H | s | H26 |

**Synthese von Ligand 3**

Umsetzung von tert-Butyl-(3,3'-di-tert-butyl-2'-((dichlorophosphino)oxy)-5,5'-dimethoxy-[1,1'-biphenyl]-2-yl)-carbonat mit 2,2'-Biphenol

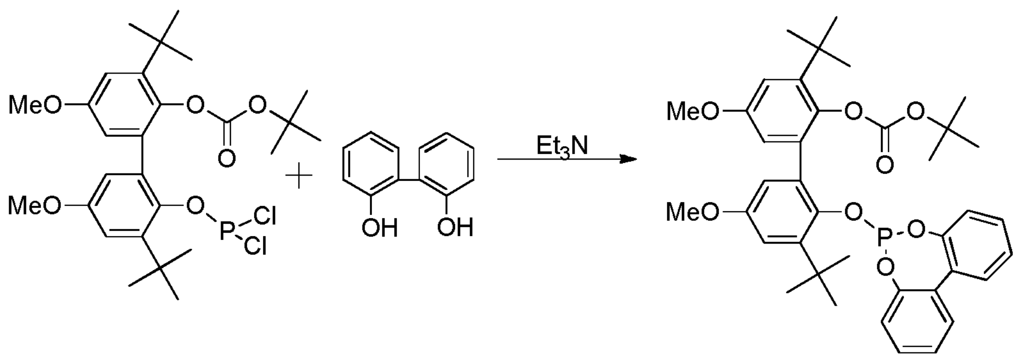


In einem sekurierten 250 ml Schlenkkolben wurden 10,5 g (0,019 mol) tert-Butyl-(3,3'-di-tert-butyl-2'-((dichlorophosphino)oxy)-5,5'-dimethoxy-[1,1'-biphenyl]-2-yl)-carbonat unter Rühren in 50 ml entgastem Acetonitril gelöst.

In einem zweiten sekurierten Schlenkkolben (250 ml) wurden 3,6 g (0,019 mol) 2,2'-Biphenol in 40 ml entgastem Acetonitril und 6,3 ml (0,045 mol) getrocknetem Triethylamin unter Rühren aufgelöst. Anschließend wurde zu der Biphenol-Triethylamin-Lösung langsam bei Raumtemperatur die Chlorophosphit-Mischung hinzugetropft und über Nacht bei Raumtemperatur gerührt. Der erhaltene Feststoff wurde filtriert und getrocknet. Das Zielprodukt konnte als weißer Feststoff (11,5 g, 90%) erhalten werden. $^{31}$P-NMR (202,4 MHz, Toluol-$d_8$): 146,2 ppm (100%).

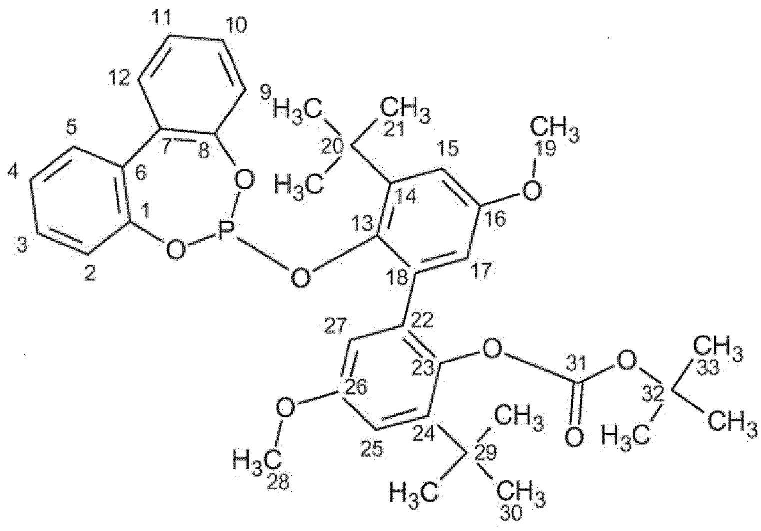

(**3**)

Tabelle 5: Zuordnung der $^{13}$C chemischen Verschiebungen von 3

| δ [ppm] | Intensität | Gruppe | Zuordnung |
|---|---|---|---|
| 157,46 | 1xC | $C_q$ | C16 |
| 155,51 | 1xC | $C_q$ | C26 |
| 151,76 | 1xC | $C_q$ | C31 |
| 150,51/149,92 | je 1 x C | $C_q$ | C1 + C8 |
| 144,07 | 1xC | $C_q$ | C24 |
| 143,62 | 1xC | $C_q$ | C13 |
| 142,70 | 1xC | $C_q$ | C23 |
| 142,67 | 1xC | $C_q$ | C14 |
| 135,75 | 1xC | $C_q$ | C18 |
| 132,13/131,73 | 1xC | $C_q$ | C6 + C7 |
| 131,30 | 1xC | $C_q$ | C22 |
| 129,87/128,94 | je 1 x C | CH | C5 + C12 |
| 128,98/128,91 | je 1 x C | CH | C3+C10 |
| 125,16/124,98 | je1xC | CH | C4+C11 |
| 123,54/122,40 | je 1 x C | CH | C2 + C9 |
| 115,14 | 1xC | CH | C25 |
| 114,93 | 1xC | CH | C15 |
| 114,85 | 1xC | CH | C27 |
| 114,72 | 1xC | CH | C17 |

(fortgesetzt)

| δ [ppm] | Intensität | Gruppe | Zuordnung |
|---|---|---|---|
| 81,36 | 1xC | $C_q$ | C32 |
| 55,14 | 1xC | $CH_3$ | C28 |
| 55,10 | 1xC | $CH_3$ | C19 |
| 35,57 | 1xC | $C_q$ | C29 |
| 35,22 | 1xC | $C_q$ | C20 |
| 30,78 | 3xC | $CH_3$ | C30 |
| 30,62 | 3xC | $CH_3$ | C21 |
| 27,45 | 3 x C | $CH_3$ | C33 |

Tabelle 6: Zuordnung der $^{1}H$ chemischen Verschiebungen von 3

| δ [ppm] | Intensität | Multiplizität | Zuordnung |
|---|---|---|---|
| 7,26 | 1H | d (J=7,7Hz) | H2,H9,H3,H10 H5,H12,H15,H25 |
| 7,15-7,09 | 6H | m | |
| 7,02 | 1H | t (J=7,7Hz) | |
| 6,95/6,91 | 2x1H | 2 x t (J~7,5Hz) | H4/H 11 |
| 6,93 | 1H | d (J=3 Hz) | H17 |
| 6,87 | 1H | d (J=3 Hz) | H27 |
| 3,48 | 3H | s | H19 |
| 3,42 | 3H | s | H29 |
| 1,50 | 9H | s | H20 |
| 1,35 | 9H | s | H21 |
| 1,16 | 9H | s | H33 |

**Synthese von Ligand 4**

Umsetzung von tert-Butyl-(3,3'-di-tert-butyl-2'-((dichlorophosphino)oxy)-5,5'-dimethoxy-[1,1'-biphenyl]-2-yl)-carbonat mit 3,3,5,5-Tetra-tert.-butyl-biphenol

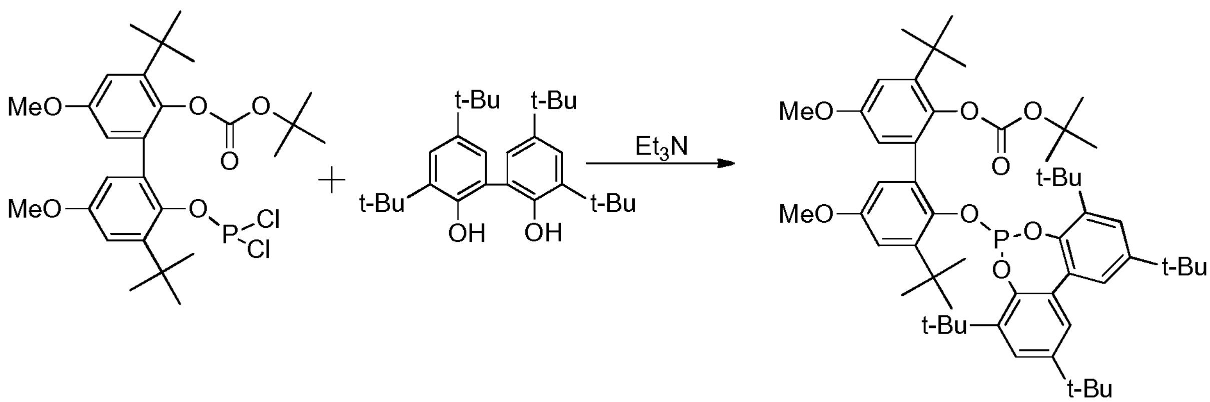

In einem sekurierten 250 ml Schlenkkolben wurden 7.0 g (0,0125 mol) tert-Butyl-(3,3'-di-tert-butyl-2'-((dichlorophosphino)oxy)-5,5'-dimethoxy-[1,1'-biphenyl]-2-yl)-carbonat in 100 ml getrocknetem Acetonitril gelöst.

In einem zweiten sekurierten Schlenkkolben (100 ml) wurden 5.1 g (0,0125 mol) 3,3',5,5'-Tetratert.-butyl-biphenol in 60 ml getrocknetem Acetonitril und 4.2 ml (0,03 mol) getrocknetem Triethylamin unter Rühren aufgelöst. Im Anschluss wurde die Biphenol-Triethylamin-Lösung langsam bei Raumtemperatur zu der Chlorophosphit-Lösung getropft und über Nacht gerührt. Ein Teil des Lösungsmittels wurde unter vermindertem Druck entfernt. Der ausgefallene Feststoff wurde filtriert und getrocknet. Das Zielprodukt konnte als weißer Feststoff (10,2 g, 91%) erhalten werden. $^{31}P$-NMR (202,4 MHz, toluene-$d_8$): 142,7 ppm (100%).

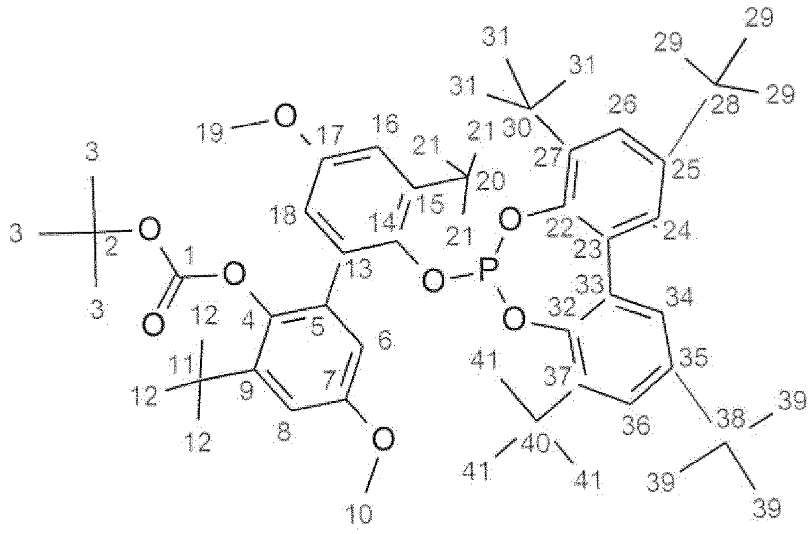

(**4**)

Tabelle 7: Zuordnung der chemischen Verschiebungen von **4**

| Nummer | δ-$^1$H / ppm | δ-$^{13}$C / ppm |
|---|---|---|
| 1 | - | 151,6 |
| 2 | - | 81,5 |
| 3 | 1,18 | 27,3 |
| 4 | - | 142,3 |
| 5 | - | 129,9 |
| 6\|18 | 6,76<br>6,67 | 114,7<br>114,1 |
| 7\|17 | - | 154,4<br>156,8 |
| 8\|16 | 6,93<br>6,88 | 113,5<br>115,4 |
| 9 | - | 143,6 |
| 10\|19 | 3,78<br>3,74 | 55,4<br>55,7 |
| 11 | - | 35,0 |
| 12 | 1,39 | 30,6 |

(fortgesetzt)

| Nummer | δ-¹H / ppm | δ-¹³C / ppm |
|---|---|---|
| 21\|29\|31\|39\|41 | 1,03<br>1,24<br>1,29<br>1,34<br>1,39<br>1,48 | 31,2<br>31,0<br>31,4<br>31,5<br>30,7<br>31,3 |
| 13 | - | 136,4 |
| 14 | - | 142,1 |
| 15 | - | 140,1 |
| 20 | - | 34,6 |
| 22\|32 | - | 146,0<br>146,9 |
| 24\|34 | 7,06<br>7,30 | 126,6<br>123,8 |
| 25135 | - | 145,1<br>146,3 |
| 26\|36 | 7,08<br>7,42 | 126,3<br>124,1 |
| 28\|38 | - | 35,1<br>35,2 |
| 30\|40 | - | 34,6<br>35,4 |
| 23\|33 | - | 135,5<br>132,6 |
| 27\|37 | - | 140,0<br>141,9 |

**Synthese von Ligand 5**

Umsetzung von tert-Butyl-3,3'-di-tert-butyl-2'-((dichlorophosphino)oxy)-5,5'-dimethoxy-[1,1'-biphenyl]-2-yl)-carbonat mit 3,3-Di-tert.butyl-5.5-dimethoxy-biphenol

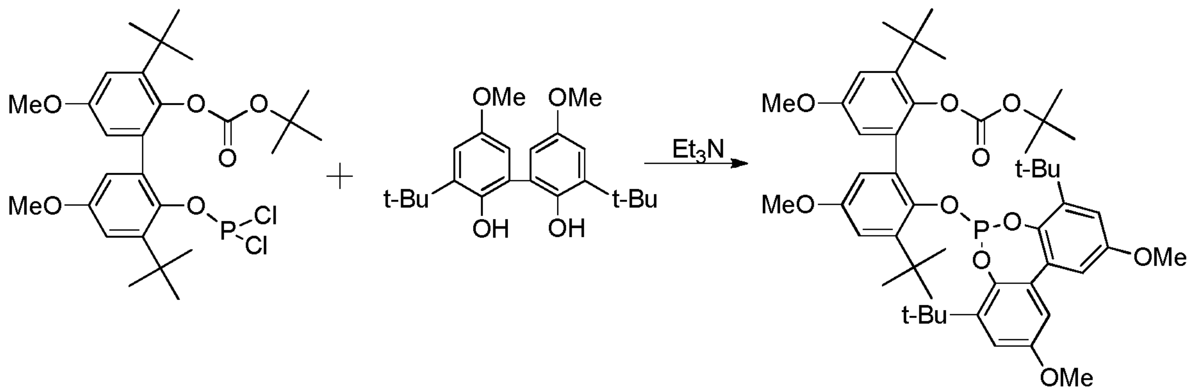

In einem sekurierten 250 ml Schlenkkolben wurden 7 g (0,0125 mol) tert-Butyl-(3,3'-di-tert-butyl-2'-((dichlorophosphino)oxy)-5,5'-dimethoxy-[1,1'-biphenyl]-2-yl)-carbonat in 100 ml getrocknetem Acetonitril gelöst.

In einem zweiten sekurierten Schlenkkolben (100 ml) wurden 4.5 g (0,0125 mol) 3,3-Di-tert.-butyl-5,5-dimethoxy-biphenol in 60 ml getrocknetem Acetonitril und 4.2 ml (0,03 mol) entgastem Triethylamin gelöst. Im Anschluss wurde die Biphenol-Triethylamin-Lösung langsam bei Raumtemperatur zu der Chlorophosphit-Lösung getropft und über Nacht bei Raumtemperatur gerührt.

Ein Teil des Lösungsmittels wurde unter vermindertem Druck entfernt. Der ausgefallene Feststoff wurde filtriert und getrocknet. Das Zielprodukt konnte als weißer Feststoff (10,5 g, 96%) erhalten werden. $^{31}$P-NMR (202,4 MHz, Toluol-$d_8$): 140,9 (95,2%) und weitere Verunreinigungen (weitere Verunreinigungen = P-H-Verbindungen, Oxidverbindungen, noch nicht vollständig umgesetztes Chlorophosphit).

<u>**Synthese von Ligand 11**</u>

<u>Umsetzung von tert-Butyl-3,3'-di-tert-butyl-2'-((dichlorophosphino)oxy)-5,5'-dimethoxy-[1,1'-biphenyl]-2-yl)-carbonat mit 2,4-Dimethylphenol</u>

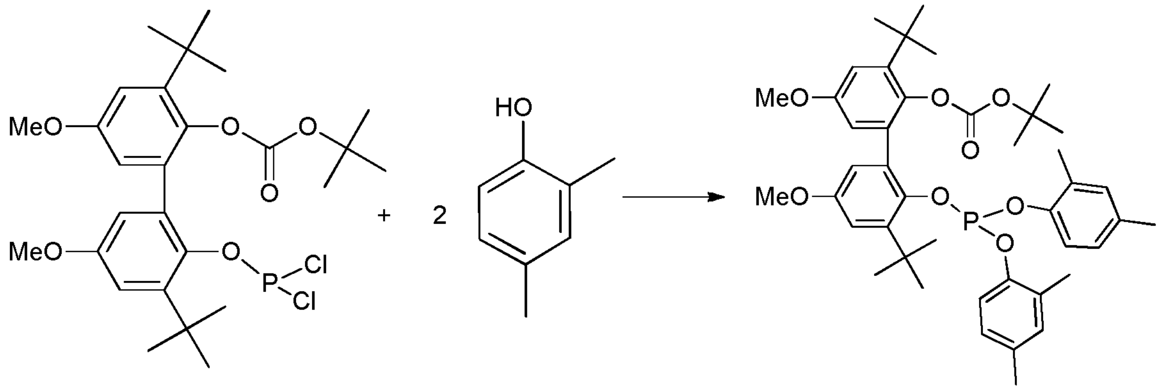

In einem sekurierten 500 ml Schlenkkolben wurden 6,8 g (0,012 mol) tert-Butyl-(3,3'-di-tert-butyl-2'-((dichlorophosphino)oxy)-5,5'-dimethoxy-[1,1'-biphenyl]-2-yl)-carbonat in 100 ml getrocknetem Acetonitril gelöst.

In einem zweiten sekurierten Schlenkkolben (250 ml) wurden 6 g (6ml; 0,048 mol) 2,4-dimethylphenol in 100 ml getrocknetem Acetonitril und 5 g (7ml; 0.059 mol) entgastem Triethylamin gelöst. Im Anschluss wurde die Biphenol-Triethylamin-Lösung langsam bei Raumtemperatur zu der Chlorophosphit-Lösung getropft und über Nacht bei Raumtemperatur gerührt und am nächsten Morgen im Eisbad gekühlt.

Ein Teil des Lösungsmittels wurde unter vermindertem Druck entfernt Hierbei entstand eine schleimartige Lösung, die nach längerem trocknen zum Feststoff wurde. Das Zielprodukt konnte als weißer Feststoff (11,8 g, 62%) erhalten werden. $^{31}$P-NMR (202,4 MHz, Toluol-$d_8$): 139,1 (92,8%) und weitere Verunreinigungen (weitere Verunreinigungen = P-H-Verbindungen, Oxidverbindungen, noch nicht vollständig umgesetztes Chlorophosphit).

**Durchführung der Katalyseversuche**

<u>Versuchsbeschreibung - allgemein</u>

In einem 100 ml-Autoklaven der Fa. Parr Instruments wurde bei verschiedenen Temperaturen, 20 und 50 bar Synthesegasdruck ($CO/H_2$ = 1:1 (Vol-%)) verschiedene Olefine hydroformyliert. Als Precursor wurden bei einer Katalysatorkonzentration von 40 ppm Rh bezogen auf das gesamte Reaktionsgemisch 0.005 g $Rh(acac)(CO)_2$ vorgelegt, bei einer Konzentration von 100 ppm Rh entsprechend 0.0123 g $Rh(acac)(CO)_2$. Als Lösemittel wurden jeweils 40 bis 46 g Toluol verwendet. Der Ligand **1** wurde in unterschiedlichen molaren Überschüssen relativ zum Rhodium verwendet. Zusätzlich wurden als GC-Standard ca. 0.5 g Tetraisopropylbenzol (TIPB) zugefügt. Ca. 6 g Edukt wurden nach Erreichen der vorgesehenen Reaktionstemperatur zudosiert.

Während der Reaktion wurde der Druck über eine Synthesegasdosierung mit Massedurchflussmesser und Druckregler konstant gehalten. Die Rührerdrehzahl betrug 1200 $min^{-1}$. Proben wurden aus der Reaktionsmischung nach 12 Stunden gezogen. Die Ergebnisse der Versuche sind in Tabelle 8 zusammengefasst (Ausbeute = Aldehyd und Alkohol Gesamtausbeute; S = Selektivität zum linearen Produkt).

(acac = acetylacetonat)

Tabelle 8:

| Eintrag | Edukt | p in [bar] | cRh in ppm | P: Rh | Ausbeute in % | S (n-Aldehyd) in % |
|---|---|---|---|---|---|---|
| 1 | cis-2-Buten | 20 | 40 | 4 | 58,0 | 45,3 |
| 2 | cis-2-Buten | 50 | 40 | 4 | 96,5 | 40,7 |
| 3 | 1-Buten | 20 | 40 | 4 | 49,2 | 51,9 |
| 4 | 1-Buten | 50 | 40 | 2 | 97,4 | 46,8 |
| 5 | 1-Buten | 50 | 92 | 8 | 99,5 | 50,3 |
| 6 | 1-Octen | 50 | 40 | 9 | 97,1 | 43,2 |
| Reaktionsbedingungen: Ligand **1,** Reaktionstemperatur: 120 °C | | | | | | |

In den Zeilen 1 und 2 sind Versuche zur rhodiumkatalysierten Hydroformylierung des Edukts cis-2-Buten mit dem Liganden **1** gelistet. Bei einem Druck von 20 bar Synthesegas werden in Versuch 1 58,0 mol-% Pentanal mit einer Pentanalselektivität von 45,3 % gebildet. Der Anteil der Hydrierung zum Alkan ist mit ca. 1.3 bis 1.5 % niedrig. Die Bildung von Pentanol wird nicht beobachtet. Eine Erhöhung des Synthesegasdruckes führt zu einer Steigerung der Pentanalausbeute auf 95.1 mol-%, die Regioselektivität fällt jedoch auf ca. 40 %.

In den Zeilen 3 bis 5 sind Hydroformylierungen von 1-Buten gelistet. Bei 20 bar (Zeile 3) wird bei einem Ligandenüberschuss von 4:1 eine Ausbeute von ca. 49 % und eine Selektivität zum n-Pentanal von ca, 52 % erreicht. Man erhält bei 50 bar Synthesegasdruck eine n-Pentanalselektivität von 50 % bei nahezu vollständigem Umsatz (Zeile 4 und 5). Die Alkanbildung ist niedrig. Im Versuch in Zeile 4 wurde der molare Überschuss des Liganden auf ca. 2:1 vermindert. Die Ausbeute steigt auf 97.4 %, die Pentanalselektivität fällt leicht auf 46.8 %. In Zeile 5 wurde die Rh-Konzentration auf ca. 100 ppm erhöht und der Ligandüberschuss auf 8:1 gegenüber Rhodium erhöht. Die Aldehydausbeute steigt auf 99.5 %, die Regioselektivtät steigt ebenfalls auf 50.3 %.

Auch längerkettige Olefine wie 1-Octen lassen sich mit guter n-Selektivität des Produktes hydroformylieren (Zeile 6).

Tabelle 9 enthält Versuche zur Hydroformylierung eines n-Octengemischs mit ca. 2 % 1-Octen, 40 % 2-Octenen, 36 % 3-Octenen und 23 % 4-Octenen (Ausbeute = Aldehyd und Alkohol Gesamtausbeute; S = Selektivität zum linearen Produkt n-Pentanal). Hierbei werden die Versuche sowohl mit dem erfindungsgemäßen Liganden 1 als auch mit dem Vergleichsliganden TDTBPP durchgeführt.

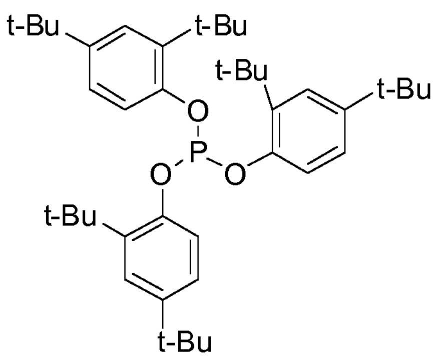

Tris(2,4-di-tert-butylphenyl)phosphit (TDTBPP)

Tabelle 9:

| Eintrag | T in [°C] | cRh in ppm | P:Rh | Ausbeute in % | S (lineares Produkt) in % |
|---|---|---|---|---|---|
| 1[a] | 80 | 90 | 20 | 99,0 | 7,2 |
| 2[a] | 80 | 90 | 5 | 99,0 | 11,1 |

(fortgesetzt)

| Eintrag | T in [°C] | cRh in ppm | P:Rh | Ausbeute in % | S (lineares Produkt) in % |
|---|---|---|---|---|---|
| 3[a] | 90 | 90 | 20 | 99,2 | 10,6 |
| 4[b] | 90 | 280 | 20 | 97,9 | 4,7 |
| 5[a] | 100 | 50 | 20 | 99,0 | 15,4 |
| 6[b] | 100 | 90 | 20 | 96,4 | 6,9 |
| 7[a] | 110 | 90 | 20 | 99,2 | 16,5 |
| 8[b] | 110 | 90 | 20 | 99,0 | 9,7 |
| 9[a] | 120 | 90 | 20 | 95,2 | 22,9 |
| 10[b] | 120 | 90 | 20 | 99,0 | 14,4 |
| 11[a] | 130 | 90 | 20 | 98,0 | 30,5 |
| 12[b] | 130 | 76 | 20 | 98,8 | 23,9 |
| 13[a] | 140 | 90 | 20 | 97,4 | 31,6 |
| 14[b] | 140 | 56 | 20 | 98,0 | 23,9 |
| 15[a] | 120 | 90 | 4 | 98,9 | 28,8 |
| 16[a] | 120 | 40 | 9,5 | 98,6 | 29,1 |
| 17[a] | 120 | 40 | 20 | 99,1 | 28,2 |
| Reaktionsbedingungen: 50 bar Synthesegas ($CO/H_2$) Substrat: n-Octene ;<br>a): Ligand 1; b): Ligand TDTBPP. | | | | | |

Die Einträge 1 bis 14 zeigen jeweils Ergebnisse von Versuchen bei konstanter Temperatur im Bereich von 80 °C bis 140 °C, und zwar jeweils für den erfindungsgemäßen Liganden 1 als auch für den Vergleichsliganden (TDTBPP). Wie deutlich zu erkennen ist, zeichnet sich der erfindungsgemäße Ligand 1 in allen Fällen durch eine deutlich höhere n-Selektivität zum gewünschten Produkt aus bei sehr guten Gesamtausbeuten. Die Bildung von Alkanen und Alkoholen ist unbedeutend. Vergleicht man die Einträge 13 und 14, so zeigt der Ligand 1 eine um knapp 8 % erhöhte Selektivität im Vergleich zu dem kommerziell verfügbaren Vergleichsliganden. Einträge 15 bis 17 zeigen die Verwendung des Liganden 1 mit unterschiedlichen molaren Überschüssen zu Rhodium. In allen Fällen konnten sehr gute n-Selektivitäten erzielt werden.

In Tabelle 10 sind die Ergebnisse für die Hydroformylierung von Di-n-Buten angegeben. Di-n-Buten ist ein Gemisch aus Isomeren von n-Octenen (ca. 16 %), 3-Methyl-heptenen (ca. 65 %) und 3,4-Dimethylhexenen (ca. 19 %) (Ausbeute = Aldehyd und Alkohol Gesamtausbeute; S = Selektivität zum linearen Produkt).

Tabelle 10:

| Eintrag | T in [°C] | p in [bar] | cRh in ppm | P:Rh | Ausbeute in % | S (nl)* in % |
|---|---|---|---|---|---|---|
| 1 | 120 | 50 | 40 | 10 | 85,1 | 30,8 |
| 2 | 120 | 50 | 90 | 7 | 89,8 | 28,3 |
| 3 | 120 | 50 | 90 | 7 | 91,5 | 30,9 |
| 4 | 120 | 50 | 100 | 5 | 93,0 | 36,5 |
| 5 | 120 | 50 | 75 | 20 | 94,6 | 29,8 |
| 6 | 130 | 50 | 75 | 20 | 95,3 | 32,7 |
| 7 | 110 | 50 | 75 | 20 | 91,9 | 24,0 |
| 8 | 120 | 40 | 75 | 20 | 94,8 | 29,0 |
| Reaktionsbedingungen: Ligand **1;** Substrat: Di-n-buten<br>* Anteil der durch endständige Oxierung entstandenen Aldehyde (im Wesentlichen Nonanal, 4-Methyloctanal, 3-Ethylheptanal, 6-Methyloctanal, 4,5-Dimethylheptanal und 3-Ethyl-4-methyl-hexanal) | | | | | | |

Die obige Tabelle 10 enthält Versuchsergebnisse für die rhodiumkatalysierte Hydroformylierung von Di-n-Buten mit dem Liganden **1.** Einträge 1 bis 5 wurden bei 120 °C und 50 bar durchgeführt, Eintrag 8 bei 40 bar. Einträge 2 und 3 sind eine Doppelbestimmmung bei einem Ligandüberschuss von 7 zu Rhodium und einer Rhodiumkonzentration bezogen auf das gesamte Reaktionsgemisch von 80 ppm, Eintrag 1 wurde bei einem Überschuss von ca. 10:1 und einer Rh-Konentration von 40 ppm ausgeführt. Eintrag 4 beschreibt eine Verminderung des Überschusses auf 5:1 gegenüber Einträgen 2 und 3. Die Einträge 5 bis 8 sind Versuche mit hohem Ligandüberschuss von ca. 20:1. Einträge 5 bis 7 wurden bei unterschiedlichen Temperaturen durchgeführt. Eintrag 8 unterscheidet sich von Eintrag 5 durch den Druck. Analog zu den Versuchen mit dem n-Octengemisch zeigen sich in den Versuchen 1 bis 6 und 8 durchweg hohe n-Selektivitäten zwischen 28 und 36 mol-%. Bei niedrigerer Temperatur (Eintrag 7) vermindert sich die n-Selektivität auf ca. 24 %. N-Octene werden in der Versuchsdauer von 12 Stunden nahezu quantitativ umgesetzt, der Umsatz der 3-Methyl-Heptene beträgt > 96 %. Die 3,4-Dimethylhexene werden zu 73-86 % umgesetzt. Somit konnte mit Hilfe der obigen Beispiele gezeigt werden, dass das neue Katalysatorsystem auch zur Oxierung von technischen Olefingemischen, die hauptsächlich verzweigte Olefine mit innenständigen Doppelbindungen enthalten, geeignet ist und ein hoher Anteil an gewünschten endständig hydroformylierten Produkten erhalten werden kann.

In Tabelle 11 sind die Ergebnisse für die Hydroformylierung von n-Octenen mit dem Liganden **3** angegeben (Ausbeute = Aldehyd und Alkohol Gesamtausbeute; S = Selektivität zum linearen Produkt).

Tabelle 11:

| Eintrag | T in [°C] | Ausbeute In % | S (lineares Produkt) in % |
|---|---|---|---|
| 1 | 80 | 30,0 | 8,6 |
| 2 | 90 | 85,9 | 4,9 |
| 3 | 100 | 91,3 | 5,9 |
| 4 | 110 | 98,5 | 8,3 |
| 5 | 120 | 99,0 | 11,9 |
| Reaktionsbedingungen: Ligand **3;** 50 bar Synthesegas; Substrat: n-Octene; cRh = 90 ppm; L/Rh = 20; | | | |

Die obige Tabelle enthält eine Zusammenstellung der Versuchsdaten für die Hydroformylierung von n-Octenen mit dem Liganden **3** in Form einer Temperaturreihe von 80 °C bis 120 °C bei 50 bar Synthesegasdruck, einer Rh-Konzentration von ca. 100 ppm und einem Ligandüberschuss von ca. 20:1. Die n-Nonanalselektivitäten sind in dieser Versuchsreihe mit 4.9 bis 11.9 mol-% niedrig, jedoch sind die Aldehydausbeuten bei höheren Temperaturen nahezu quantitativ und sehr gut. Die Alkanbildung ist mit < 1% gering, die Hydrierung zu Alkoholen wird nicht beobachtet.

In Tabelle 12 sind die Ergebnisse für die Hydroformylierung von Di-n-Buten angegeben. Di-n-Buten ist ein Gemisch aus Isomeren von n-Octenen (ca. 16 %), 3-Methyl-heptenen (ca. 65 %) und 3,4-Dimethylhexenen (ca. 19 %) (Ausbeute = Aldehyd und Alkohol Gesamtausbeute; S = Selektivität zum linearen Produkt).

Tabelle 12:

| Eintrag | Ligand | T in [°C] | p in [bar] | cRh in ppm | P:Rh | Ausbeute in % |
|---|---|---|---|---|---|---|
| 1 | 4 | 140 | 50 | 100 | 20:1 | 38 |
| 2 | 3 | 140 | 50 | 100 | 10:1 | 74 |
| 3 | 5 | 120 | 50 | 100 | 20:1 | 85 |
| 4 | 5 | 130 | 50 | 100 | 20:1 | 86 |
| 5 | 5 | 140 | 50 | 100 | 20:1 | 96 |
| 6 | 5 | 140 | 50 | 100 | 15:1 | 97 |
| 7 | 5 | 140 | 50 | 100 | 5:1 | 97 |
| Reaktionsbedingungen: Ligand **1;** Substrat: Di-n-buten<br>* Anteil der durch endständige Oxierung entstandenen Aldehyde (im Wesentlichen Nonanal, 4-Methyloctanal, 3-Ethylheptanal, 6-Methyloctanal, 4,5-Dimethylheptanal und 3-Ethyl-4-methyl-hexanal) | | | | | | |

Die obige Tabelle 12 enthält Versuchsergebnisse für die rhodiumkatalysierte Hydroformylierung von Di-n-Buten

mit den Liganden **3, 4** und **5.** Während der Ligand **4** (Eintrag 1) eine moderate Gesamtausbeute an Aldehyden und Alkoholen liefert, zeichnen sich sie Liganden **3** und **4** durch sehr gute Ausbeuten aus. Insbesondere der Ligand **5** zeigt bei erhöhter Temperatur (140°C) nahezu quantitative Umsätze (Einträge 5 bis 7).

Somit konnte mit Hilfe der obigen Beispiele gezeigt werden, dass das neue Katalysatorsystem auch zur Oxierung von technischen Olefingemischen, die hauptsächlich verzweigte Olefine mit innenständigen Doppelbindungen enthalten, geeignet ist und ein hoher Anteil an gewünschten hydroformylierten Produkten erhalten werden kann.

Die erfindungsgemäßen Monophosphitliganden, weisen eine sehr gute n-Selektivität in Bezug auf die Hydroformylierung auf. Die Selektivität zu den gewünschten linearen Aldehyden ist hierbei deutlich größer als beispielsweise bei dem kommerziell verfügbaren Liganden TDTBPP. Die gestellten Aufgaben werden somit durch diese neuartigen, erfindungsgemäßen Liganden gelöst.

## Patentansprüche

1. Ligand, welcher eine der beiden allgemeinen Strukturen **I** oder **II** aufweist:

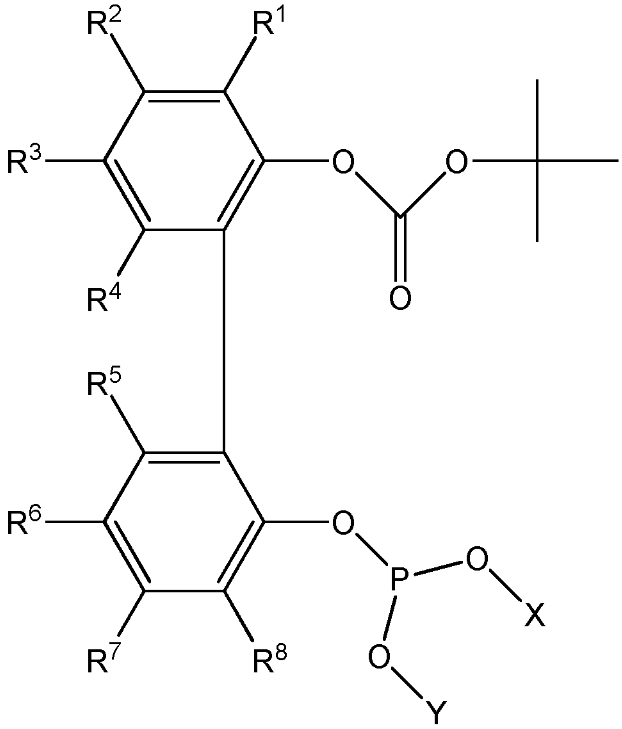

**I**

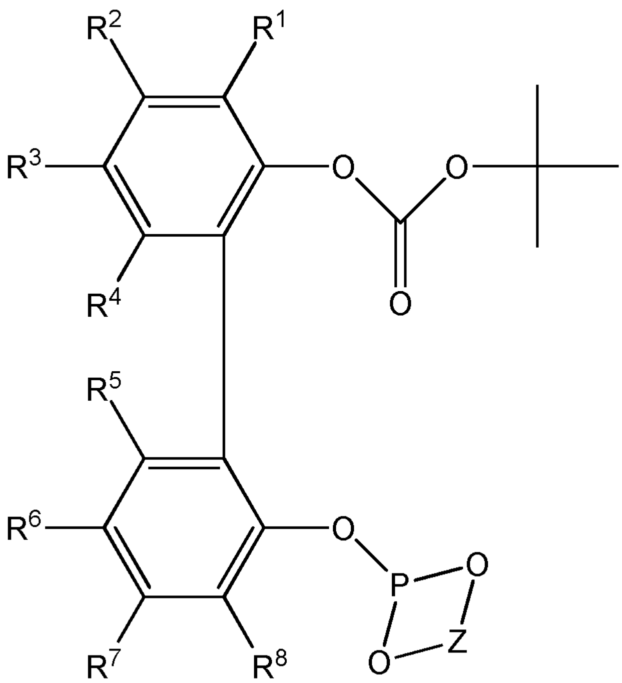

II

wobei

$R^1$, $R^2$, $R^3$, $R^4$, $R^5$, $R^6$, $R^7$, $R^8$ jeweils unabhängig voneinander ausgewählt sind aus: -H, $-(C_1-C_{12})$-Alkyl, $-O-(C_1-C_{12})$-Alkyl, $-O-(C_6-C_{20})$-Aryl, $-(C_6-C_{20})$-Aryl, Halogen, COO-$(C_1-C_{12})$-Alkyl, CONH-$(C_1-C_{12})$-Alkyl, $-(C_6-C_{20})$-Aryl-CON[$(C_1-C_{12})$-Alkyl]$_2$, -CO-$(C_1-C_{12})$-Alkyl, -CO-$(C_6-C_{20})$-Aryl,-COOH, -OH, $-SO_3H$; $-SO_3Na$, $-NO_2$, -CN, $-NH_2$, -N[$(C_1-C_{12})$-Alkyl]$_2$;

X und Y jeweils unabhängig voneinander ausgewählt sind aus:

$-(C_1-C_{12})$-Alkyl, $-(C_6-C_{20})$-Aryl, $-(C_6-C_{20})$-Aryl-$(C_1-C_{12})$-Alkyl, $-(C_6-C_{20})$-Aryl-O-$(C_1-C_{12})$-Alkyl, $-(C_1-C_{12})$-Alkyl-$(C_6-C_{20})$-Aryl, $-(C_6-C_{20})$-Aryl-COO-$(C_1-C_{12})$-Alkyl, $-(C_6-C_{20})$-Aryl-CONH-$(C_1-C_{12})$-Alkyl,$-(C_6-C_{20})$-Aryl-CON[$(C_1-C_{12})$-Alkyl]$_2$, $-(C_4-C_{20})$-Heteroaryl, $-(C_4-C_{20})$-Heteroaryl-$(C_1-C_{12})$-Alkyl,$-(C_5-C_8)$-Cycloalkyl-$(C_4-C_{20})$-Aryl-CO-$(C_6-C_{20})$-Aryl,

Z ausgewählt ist aus:

$-(C_1-C_{12})$-Alkyl-, $-(C_6-C_{20})$-Aryl-, $-(C_6-C_{20})$-Aryl-$(C_1-C_{12})$-Alkyl-, $-(C_1-C_{12})$-Alkyl-$(C_6-C_{20})$-Aryl-, $-(C_4-C_{20})$-Heteroaryl-, $-(C_6-C_{20})$-Aryl-CO-$(C_6-C_{20})$-Aryl-, $-(C_6-C_{20})$-Aryl-$(C_6-C_{20})$-Aryl-;

wobei die genannten Alkyl-, Heteroalkyl-, Cycloalkyl-, Heterocycloalkyl-, Aryl- und Heteroarylgruppen substituiert sein können.

**2.** Ligand nach Anspruch 1,
wobei X und Y jeweils unabhängig voneinander ausgewählt sind aus:

$-(C_1-C_{12})$-Alkyl, $-(C_6-C_{20})$-Aryl, $-(C_6-C_{20})$-Aryl-$(C_1-C_{12})$-Alkyl, $-(C_6-C_{20})$-Aryl-O-$(C_1-C_{12})$-Alkyl, $-(C_6-C_{20})$-Aryl-COO-$(C_1-C_{12})$-Alkyl, $-(C_6-C_{20})$-Aryl-CONH-$(C_1-C_{12})$-Alkyl, $-(C_6-C_{20})$-Aryl-CON[$(C_1-C_{12})$.-Alkyl]$_2$, $-(C_4-C_{20})$-Heteroaryl, $-(C_4-C_{20})$-Heteroaryl-$(C_1-C_{12})$-Alkyl.

**3.** Ligand nach einem der Ansprüche 1 oder 2,
wobei X und Y jeweils unabhängig voneinander ausgewählt sind aus:

$-(C_1-C_{12})$-Alkyl, $-(C_6-C_{20})$-Aryl, $-(C_6-C_{20})$-Aryl-$(C_1-C_{12})$-Alkyl, $-(C_6-C_{20})$-Aryl-O-$(C_1-C_{12})$-Alkyl, $-(C_6-C_{20})$-Aryl-

COO-($C_1$-$C_{12}$)-Alkyl.

**4.** Ligand nach einem der Ansprüche 1 bis 3,
wobei Z ausgewählt ist aus:

-($C_1$-$C_{12}$)-Alkyl-, -($C_6$-$C_{20}$)-Aryl-, -($C_6$-$C_{20}$)-Aryl-($C_1$-$C_{12}$)-Alkyl-, -($C_6$-$C_{20}$)-Aryl-CO-($C_6$-$C_{20}$)-Aryl-,-($C_1$-$C_{12}$)-Alkyl-($C_6$-$C_{20}$)-Aryl-, -($C_6$-$C_{20}$)-Aryl-($C_6$-$C_{20}$)-Aryl-.

**5.** Ligand nach einem der Ansprüche 1 bis 4,
wobei $R^1$, $R^2$, $R^3$, $R^4$, $R^5$, $R^6$, $R^7$, $R^8$ jeweils unabhängig voneinander ausgewählt sind aus: -H, - ($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl,-COOH, -OH, -$NH_2$, -N[($C_1$-$C_{12}$)-Alkyl]$_2$.

**6.** Ligand nach einem der Ansprüche 1 bis 5,
wobei X und Y für die gleichen Reste stehen.

**7.** Ligand nach einem der Ansprüche 1 bis 6,
wobei $R^3$ und $R^6$ für -O-($C_1$-$C_{12}$)-Alkyl stehen.

**8.** Ligand nach einem der Ansprüche 1 bis 7,
wobei $R^3$ und $R^6$ für -OMe stehen.

**9.** Ligand nach einem der Ansprüche 1 bis 8,
wobei $R^1$ und $R^8$ für -($C_1$-$C_{12}$)-Alkyl stehen.

**10.** Ligand nach einem der Ansprüche 1 bis 9,
wobei $R^1$ und $R^8$ für *tert*-Butyl stehen.

**11.** Ligand nach einem der Ansprüche 1 bis 10,
welcher die allgemeine Strukturen **III** aufweist:

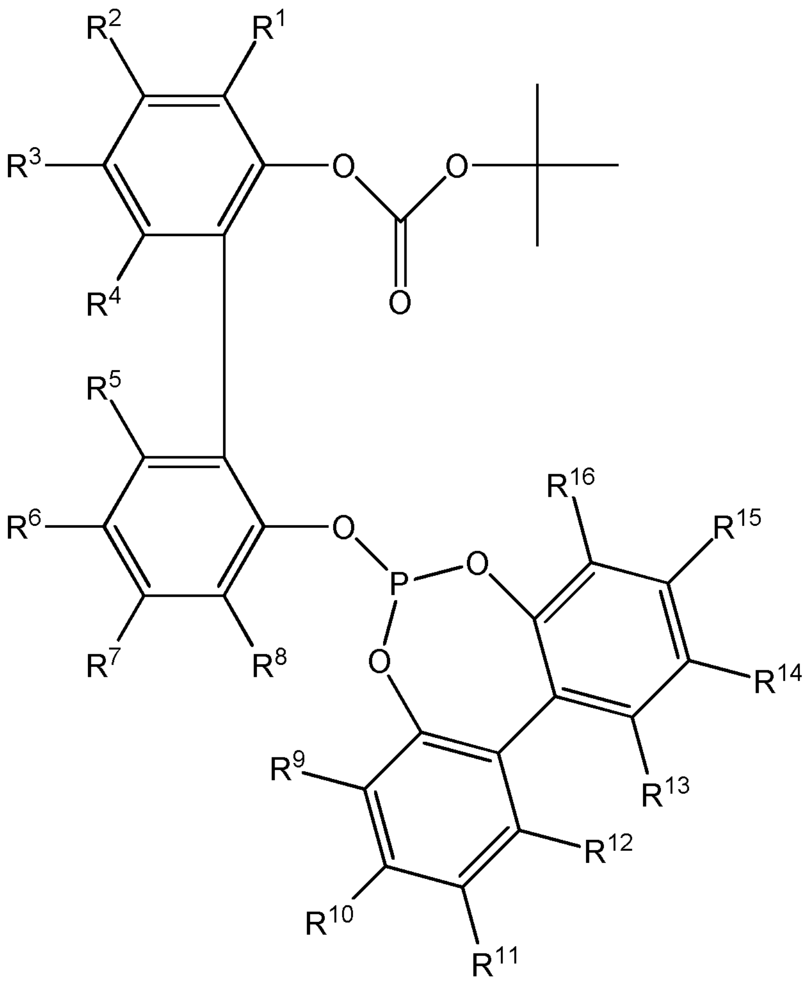

**III**

wobei $R^9$, $R^{10}$, $R^{11}$, $R^{12}$, $R^{13}$, $R^{14}$, $R^{15}$, $R^{16}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3H$; -$SO_3Na$, -$NO_2$, -CN, -$NH_2$, -N[($C_1$-$C_{12}$)-Alkyl]$_2$.

**12.** Ligand nach einem der Ansprüche 1 bis 11, welcher die allgemeine Strukturen **IV** aufweist:

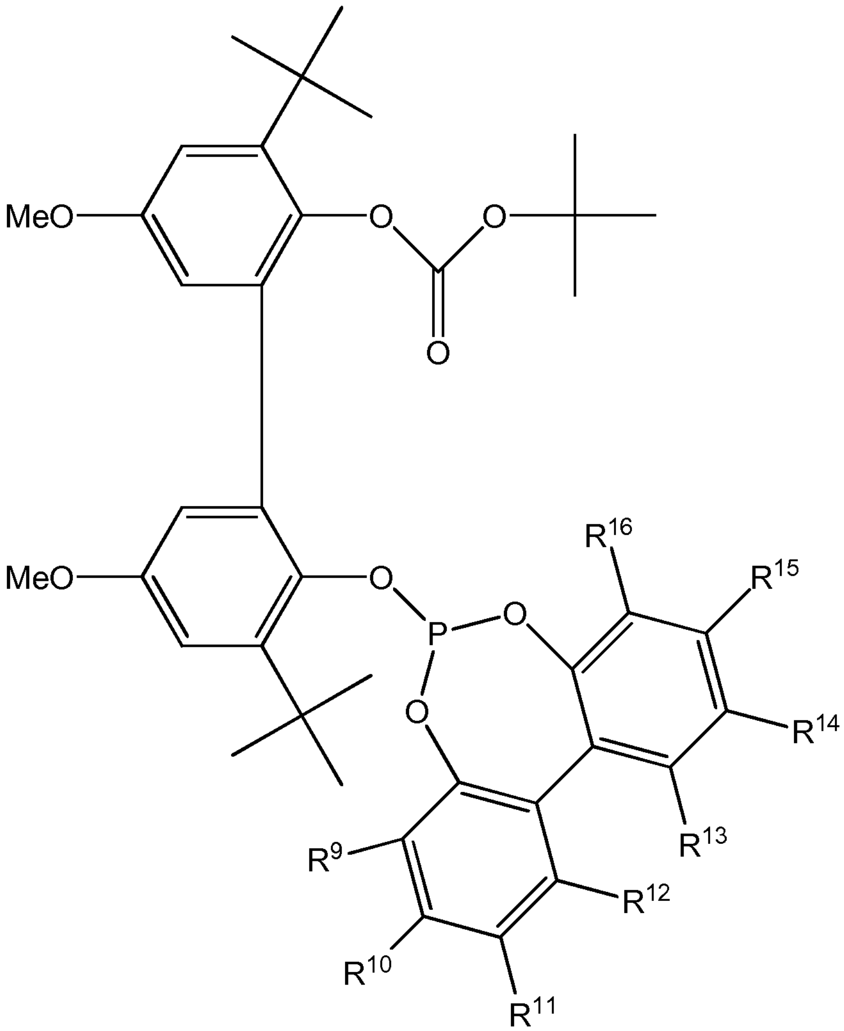

IV

wobei $R^9$, $R^{10}$, $R^{11}$, $R^{12}$, $R^{13}$, $R^{14}$, $R^{15}$, $R^{16}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, -OH, -$SO_3H$; -$SO_3Na$, -$NO_2$, -CN, -$NH_2$, -N[($C_1$-$C_{12}$)-Alkyl]$_2$.

**13.** Komplex umfassend:

- einen Liganden nach einem der Ansprüche 1 bis 12,
- ein Metallatom ausgewählt aus: Rh, Ru, Co, Ir.

**14.** Verwendung eines Liganden nach einem der Ansprüche 1 bis 12, in einem Ligand-Metall-Komplex zur Katalyse einer Hydroformylierungsreaktion.

**15.** Verfahren umfassend die Verfahrensschritte:

a) Vorlegen eines Olefins,
b) Zugabe eines Komplexes nach Anspruch 13, oder eines Liganden nach einem der Ansprüche 1 bis 12 und einer Verbindung, welche ein Metallatom ausgewählt aus: Rh, Ru, Co, Ir aufweist,
c) Zuführen von $H_2$ und CO,
d) Erwärmen des Reaktionsgemisches, wobei das Olefin zu einem Aldehyd umgesetzt wird.

## Claims

1. Ligand having one of the two general structures **I** and **II:**

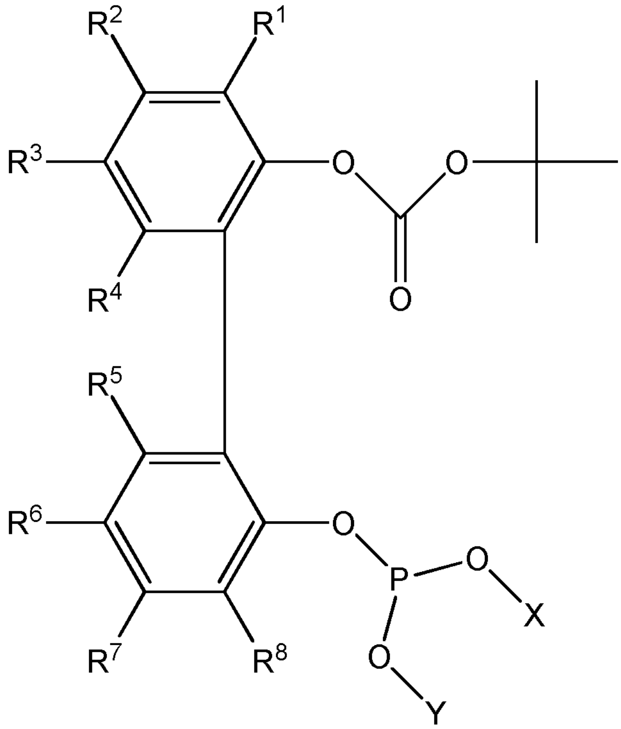

I

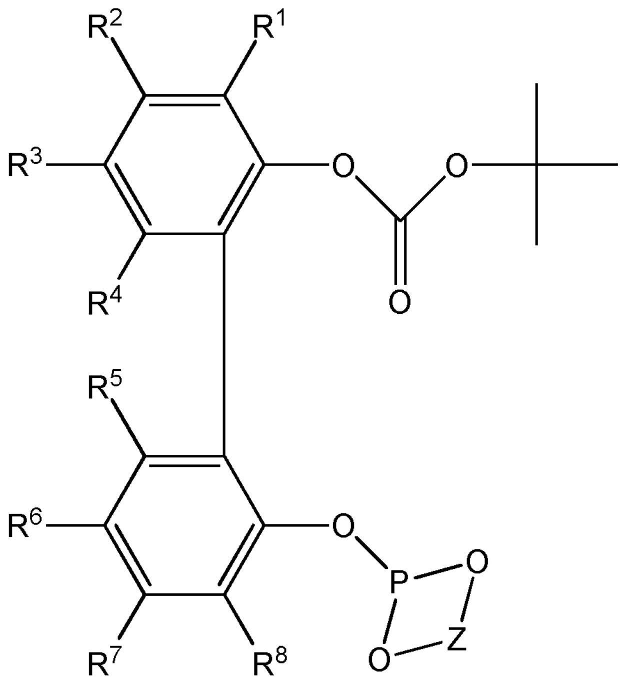

II

where
$R^1$, $R^2$, $R^3$, $R^4$, $R^5$, $R^6$, $R^7$, $R^8$ are each independently selected from: -H, -($C_1$-$C_{12}$)-alkyl,

-O-($C_1$-$C_{12}$)-alkyl,-O-($C_6$-$C_{20}$)-aryl, - ($C_6$-$C_{20}$)-aryl, halogen, COO-($C_1$-$C_{12}$)-alkyl, CONH-($C_1$-$C_{12}$)-alkyl, -($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH,-OH, -$SO_3H$, -$SO_3Na$, -$NO_2$, -CN, -$NH_2$, -N[($C_1$-$C_{12}$)-alkyl]$_2$;
X and Y are each independently selected from:

-($C_1$-$C_{12}$)-alkyl, -($C_6$-$C_{20}$)-aryl, -($C_6$-$C_{20}$)-aryl-($C_1$-$C_{12}$)-alkyl, -($C_6$-$C_{20}$)-aryl-O-($C_1$-$C_{12}$)-alkyl, -($C_1$-$C_{12}$)-alkyl-($C_6$-$C_{20}$)-aryl, -($C_6$-$C_{20}$) -aryl-COO-($C_1$-$C_{12}$)-alkyl, - ($C_6$-$C_{20}$)-aryl-CONH-($C_1$-$C_{12}$)-alkyl, -($C_6$-$C_{20}$)-aryl-CON[($C_1$-$C_{12}$)-alkyl]$_2$, -($C_4$-$C_{20}$)-heteroaryl, -($C_4$-$C_{20}$)-heteroaryl-($C_1$-$C_{12}$)-alkyl, -($C_5$-$C_8$)-Cycloalkyl-($C_4$-$C_{20}$)-aryl-CO-($C_6$-$C_{20}$)-aryl,

Z is selected from:

-($C_1$-$C_{12}$)-alkyl-, -($C_6$-$C_{20}$)-aryl-, -($C_6$-$C_{20}$)-aryl-($C_1$-$C_{12}$)-alkyl-, - ($C_1$-$C_{12}$)-alkyl-($C_6$-$C_{20}$)-aryl-, - ($C_4$-$C_{20}$)-heteroaryl-, -($C_6$-$C_{20}$)-aryl-CO-($C_6$-$C_{20}$)-aryl-, -($C_6$-$C_{20}$)-aryl- ($C_6$-$C_{20}$) -aryl-;

where the alkyl, heteroalkyl, cycloalkyl, heterocycloalkyl, aryl and heteroaryl groups mentioned may be substituted.

**2.** Ligand according to Claim 1,
where X and Y are each independently selected from:

-($C_1$-$C_{12}$)-alkyl, -($C_6$-$C_{20}$)-aryl, -($C_6$-$C_{20}$)-aryl-($C_1$-$C_{12}$)-alkyl, -($C_6$-$C_{20}$)-aryl-O-($C_1$-$C_{12}$)-alkyl, - ($C_6$-$C_{20}$)-aryl-COO-($C_1$-$C_{12}$)-alkyl, -($C_6$-$C_{20}$)-aryl-CONH-($C_1$-$C_{12}$)-alkyl,-($C_6$-$C_{20}$)-aryl-CON[($C_1$-$C_{12}$)-alkyl]$_2$, -($C_4$-$C_{20}$)-heteroaryl, - ($C_4$-$C_{20}$) -heteroaryl- ($C_1$-$C_{12}$)-alkyl.

**3.** Ligand according to either of Claims 1 and 2, where X and Y are each independently selected from:

- ($C_1$-$C_{12}$) -alkyl, - ($C_6$-$C_{20}$) -aryl, -($C_6$-$C_{20}$)-aryl-($C_1$-$C_{12}$)-alkyl, - ($C_6$-$C_{20}$)-aryl-O-($C_1$-$C_{12}$)-alkyl, ($C_6$-$C_{20}$)-aryl-COO-($C_1$-$C_{12}$)-alkyl.

**4.** Ligand according to any of Claims 1 to 3,
where Z is selected from:

-($C_1$-$C_{12}$)-alkyl-, -($C_6$-$C_{20}$)-aryl-, -($C_6$-$C_{20}$)-aryl-($C_1$-$C_{12}$)-alkyl-, -($C_6$-$C_{20}$-aryl-CO-($C_6$-$C_{20}$)-aryl-, -($C_1$-$C_{12}$)-alkyl-($C_6$-$C_{20}$)-aryl-, -($C_6$-$C_{20}$)-aryl- ($C_6$-$C_{20}$)-aryl-.

**5.** Ligand according to any of Claims 1 to 4,
where $R^1$, $R^2$, $R^3$, $R^4$, $R^5$, $R^6$, $R^7$, $R^8$ are each independently selected from: -H, -($C_1$-$C_{12}$)-alkyl, -O-($C_1$-$C_{12}$)-alkyl, -O-($C_6$-$C_{20}$)-aryl, ($C_6$-$C_{20}$)-aryl, -COO-($C_1$-$C_{12}$)-alkyl, -CONH-($C_1$-$C_{12}$)-alkyl, -($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$NH_2$, -N[($C_1$-$C_{12}$)-alkyl]$_2$.

**6.** Ligand according to any of Claims 1 to 5,
where X and Y are the same radicals.

**7.** Ligand according to any of Claims 1 to 6,
where $R^3$ and $R^6$ are each -O-($C_1$-$C_{12}$) -alkyl.

**8.** Ligand according to any of Claims 1 to 7,
where $R^3$ and $R^6$ are each -OMe.

**9.** Ligand according to any of Claims 1 to 8,
where $R^1$ and $R^8$ are each - ($C_1$-$C_{12}$)-alkyl .

**10.** Ligand according to any of Claims 1 to 9,
where $R^1$ and $R^8$ are each *tert*-butyl.

**11.** Ligand according to any of Claims 1 to 10,
having the general structures III:

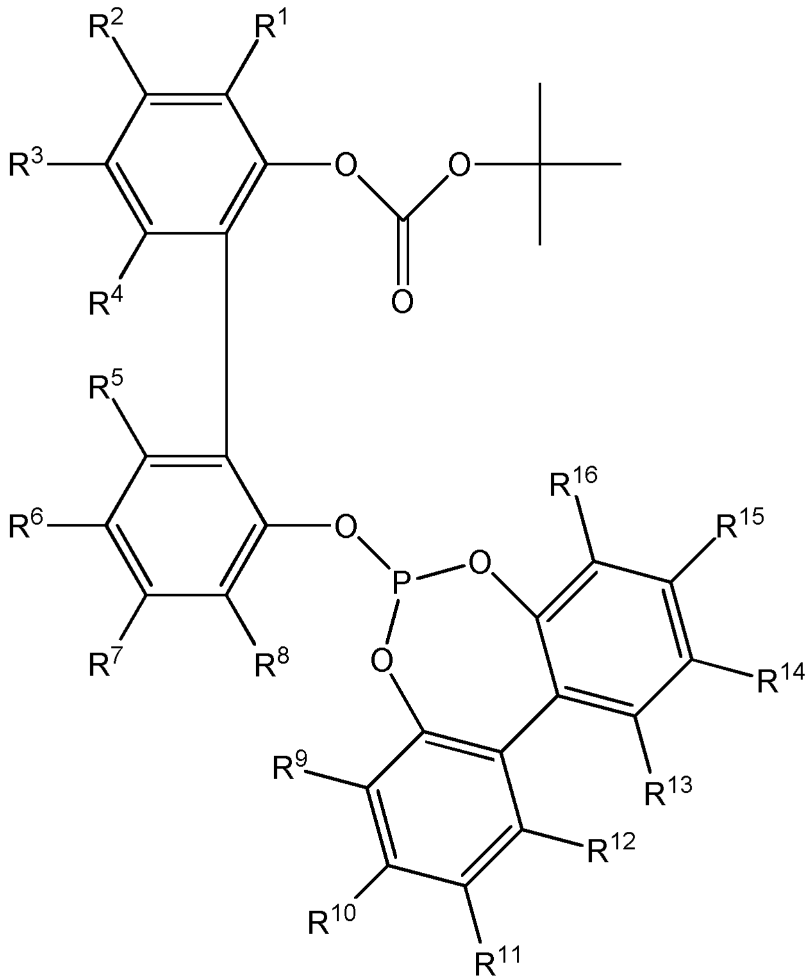

III

where $R^9$, $R^{10}$, $R^{11}$, $R^{12}$, $R^{13}$, $R^{14}$, $R^{15}$, $R^{16}$ are each independently selected from:

-H, $-(C_1-C_{12})$-alkyl, $-O-(C_1-C_{12})$-alkyl, $-O-(C_6-C_{20})$-aryl, $-(C_6-C_{20})$-aryl, -halogen, $-COO-(C_1-C_{12})$-alkyl, $-CONH-(C_1-C_{12})$-alkyl, $-(C_6-C_{20})$-aryl-$CON[(C_1-C_{12})$-alkyl$]_2$, $-CO-(C_1-C_{12})$-alkyl, $-CO-(C_6-C_{20})$-aryl, -COOH, -OH, $-SO_3H$, $-SO_3Na$, $-NO_2$, -CN, $-NH_2$, $-N[(C_1-C_{12})$-alkyl$]_2$.

**12.** Ligand according to any of Claims 1 to 11, having the general structures IV:

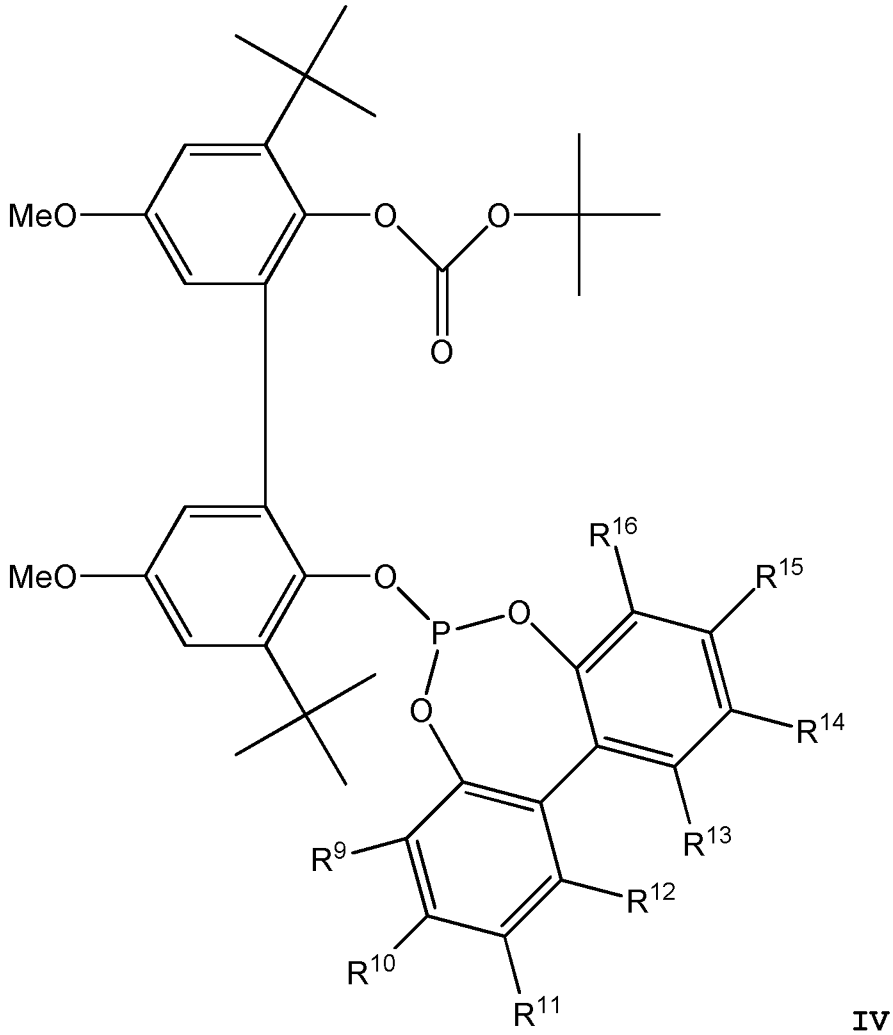

IV

where $R^9$, $R^{10}$, $R^{11}$, $R^{12}$, $R^{13}$, $R^{14}$, $R^{15}$, $R^{16}$ are each independently selected from:

-H, -($C_1$-$C_{12}$)-alkyl, -O-($C_1$-$C_{12}$)-alkyl, -O-($C_6$-$C_{20}$) -aryl, -($C_6$-$C_{20}$)-aryl, -halogen, -COO-($C_1$-$C_{12}$)-alkyl, -CONH-($C_1$-$C_{12}$)-alkyl, -($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3H$, -$SO_3Na$, -$NO_2$, -CN, -$NH_2$, -N[($C_1$-$C_{12}$)-alkyl]$_2$.

**13.** Complex comprising:

- a ligand according to any of Claims 1 to 12,
- a metal atom selected from: Rh, Ru, Co, Ir.

**14.** Use of a ligand according to any of Claims 1 to 12 in a ligand-metal complex for catalysis of a hydroformylation reaction.

**15.** Process comprising the process steps of:

a) initially charging an olefin,
b) adding a complex according to Claim 13,
or a ligand according to any of Claims 1 to 12 and a compound including a metal atom selected from: Rh, Ru, Co, Ir,
c) feeding in $H_2$ and CO,
d) heating the reaction mixture, with conversion of the olefin to an aldehyde.

**Revendications**

1. Ligand, qui présente une des deux structures générales I ou II :

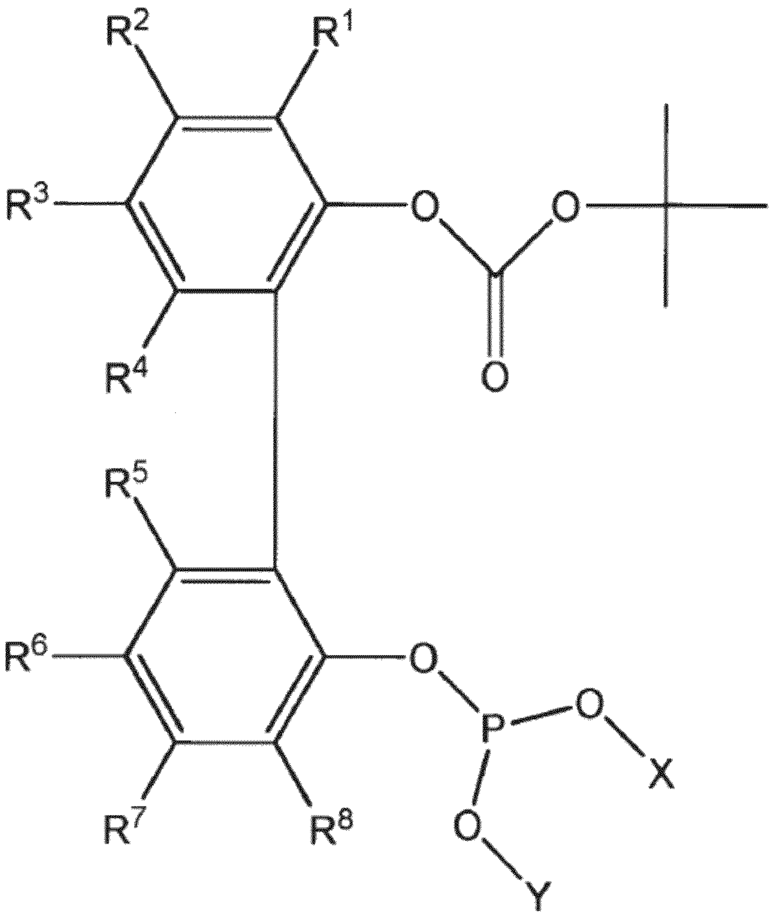

I

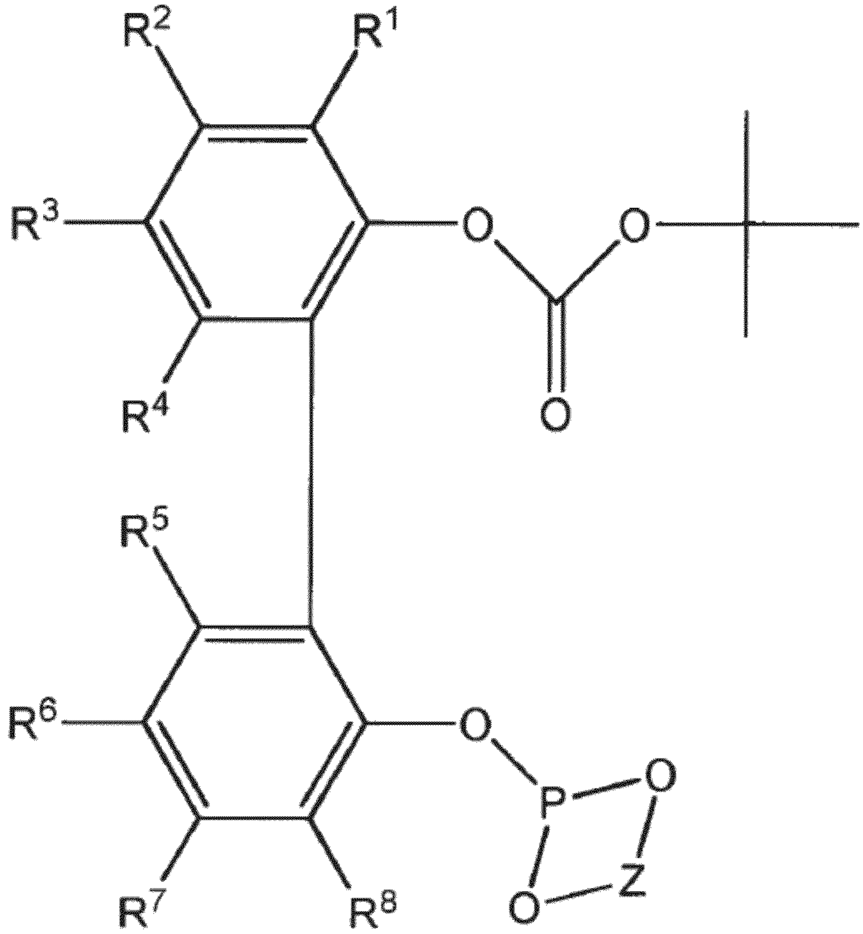

II

dans lesquelles
$R^1$, $R^2$, $R^3$, $R^4$, $R^5$, $R^6$, $R^7$, $R^8$ sont chacun choisis indépendamment les uns des autres parmi : H, -alkyle en ($C_1$-$C_{12}$), -O-alkyle en ($C_1$-$C_{12}$), -0-aryle en ($C_6$-$C_{20}$),-aryle en ($C_6$-$C_{20}$), halogène, COO-alkyle en ($C_1$-$C_{12}$), CONH-alkyle en ($C_1$-$C_{12}$), -aryle en ($C_6$-$C_{20}$)-CON[alkyle en ($C_1$-$C_{12}$)]$_2$, -CO-alkyle en ($C_1$-$C_{12}$), -CO-aryle en ($C_6$-$C_{20}$), -COOH, -OH, -$SO_3H$; -$SO_3Na$, -$NO_2$, -CN, -$NH_2$, -N[alkyle en ($C_1$-$C_{12}$]$_2$ ;
X et Y sont chacun choisis indépendamment l'un de l'autre parmi :

-alkyle en ($C_1$-$C_{12}$), -aryle en ($C_6$-$C_{20}$), -aryle en ($C_6$-$C_{20}$)-alkyle en ($C_1$-$C_{12}$), -aryle en ($C_6$-$C_{20}$)-O-alkyle en ($C_1$-$C_{12}$), -alkyle en ($C_1$-$C_{12}$) -aryle en ($C_6$-$C_{20}$), -aryle en ($C_6$-$C_{20}$)-COO-alkyle en ($C_1$-$C_{12}$), -aryle en ($C_6$-$C_{20}$)-CONH-alkyle en ($C_1$-$C_{12}$), -aryle en ($C_6$-$C_{20}$)-CON [alkyle en ($C_1$-$C_{12}$)]$_2$, -hétéroaryle en ($C_4$-$C_{20}$), -hétéroaryle en ($C_4$-$C_{20}$)-alkyle en ($C_1$-$C_{12}$), -cycloalkyle en ($C_5$-$C_8$)-aryle en ($C_4$-$C_{20}$) -CO-aryle en ($C_6$-$C_{20}$),

Z est choisi parmi :

-alkyle en ($C_1$-$C_{12}$), -aryle en ($C_6$-$C_{20}$), -aryle en ($C_6$-$C_{20}$)-alkyle en ($C_1$-$C_{12}$), -alkyle en ($C_1$-$C_{12}$) -aryle en ($C_6$-$C_{20}$), -hétéroaryle en ($C_4$-$C_{20}$), -aryle en ($C_6$-$C_{20}$)-CO-aryle en ($C_6$-$C_{20}$), -aryle en ($C_6$-$C_{20}$)-aryle en ($C_6$-$C_{20}$) ;

les groupes alkyle, hétéroalkyle, cycloalkyle, hétérocycloalkyle, aryle et hétéroaryle mentionnés pouvant être substitués.

**2.** Ligand selon la revendication 1, dans lequel X et Y sont chacun choisis indépendamment l'un de l'autre parmi :

-alkyle en ($C_1$-$C_{12}$), -aryle en ($C_6$-$C_{20}$), -aryle en ($C_6$-$C_{20}$)-alkyle en ($C_1$-$C_{12}$), -aryle en ($C_6$-$C_{20}$)-O-alkyle en ($C_1$-$C_{12}$), -aryle en ($C_6$-$C_{20}$) -COO-alkyle en ($C_1$-$C_{12}$),-aryle en ($C_6$-$C_{20}$)-CONH-alkyle en ($C_1$-$C_{12}$), -aryle en

$(C_6-C_{20})$-CON[alkyle en $(C_1-C_{12})]_2$, -hétéroaryle en $(C_4-C_{20})$,-hétéroaryle en $(C_4-C_{20})$-alkyle en $(C_1-C_{12})$.

**3.** Ligand selon l'une quelconque des revendications 1 ou 2, dans lequel X et Y sont chacun choisis indépendamment l'un de l'autre parmi :

-alkyle en $(C_1-C_{12})$, -aryle en $(C_6-C_{20})$, -aryle en $(C_6-C_{20})$-alkyle en $(C_1-C_{12})$, -aryle en $(C_6-C_{20})$-O-alkyle en $(C_1-C_{12})$, -aryle en $(C_6-C_{20})$ -COO-alkyle en $(C_1-C_{12})$.

**4.** Ligand selon l'une quelconque des revendications 1 à 3, dans lequel Z est choisi parmi :

-alkyle en $(C_1-C_{12})$, -aryle en $(C_6-C_{20})$, -aryle en $(C_6-C_{20})$-alkyle en $(C_1-C_{12})$, -aryle en $(C_6-C_{20})$-CO-aryle en $(C_6-C_{20})$, alkyle en $(C_1-C_{12})$ -aryle en $(C_6-C_{20})$, -aryle en $(C_6-C_{20})$ -aryle en $(C_6-C_{20})$.

**5.** Ligand selon l'une quelconque des revendications 1 à 4, dans lequel $R^1$, $R^2$, $R^3$, $R^4$, , $R^5$, $R^6$, $R^7$, $R^8$ sont chacun choisis indépendamment les uns des autres parmi : H, -alkyle en $(C_1-C_{12})$, -O-alkyle en $(C_1-C_{12})$,-O-aryle en $(C_6-C_{20})$, -aryle en $(C_6-C_{20})$, COO-alkyle en $(C_1-C_{12})$, -CONH-alkyle en $(C_1-C_{12})$, -aryle en $(C_6-C_{20})$-CON[alkyle en $(C_1-C_{12})]_2$, -CO-alkyle en $(C_1-C_{12})$, -CO-aryle en $(C_6-C_{20})$, -COOH, -OH, $-NH_2$, -N[alkyle en $(C_1-C_{12})]_2$.

**6.** Ligand selon l'une quelconque des revendications 1 à 5, dans lequel X et Y représentent des radicaux identiques.

**7.** Ligand selon l'une quelconque des revendications 1 à 6, dans lequel $R^3$ et $R^6$ représentent -O-alkyle en $(C_1-C_{12})$.

**8.** Ligand selon l'une quelconque des revendications 1 à 7, dans lequel $R^3$ et $R^6$ représentent -OMe.

**9.** Ligand selon l'une quelconque des revendications 1 à 8, dans lequel $R^1$ et $R^8$ représentent -alkyle en $(C_1-C_{12})$.

**10.** Ligand selon l'une quelconque des revendications 1 à 9, dans lequel $R^1$ et $R^8$ représentent *tert*-butyle.

**11.** Ligand selon l'une quelconque des revendications 1 à 10, qui présente les structures générale III :

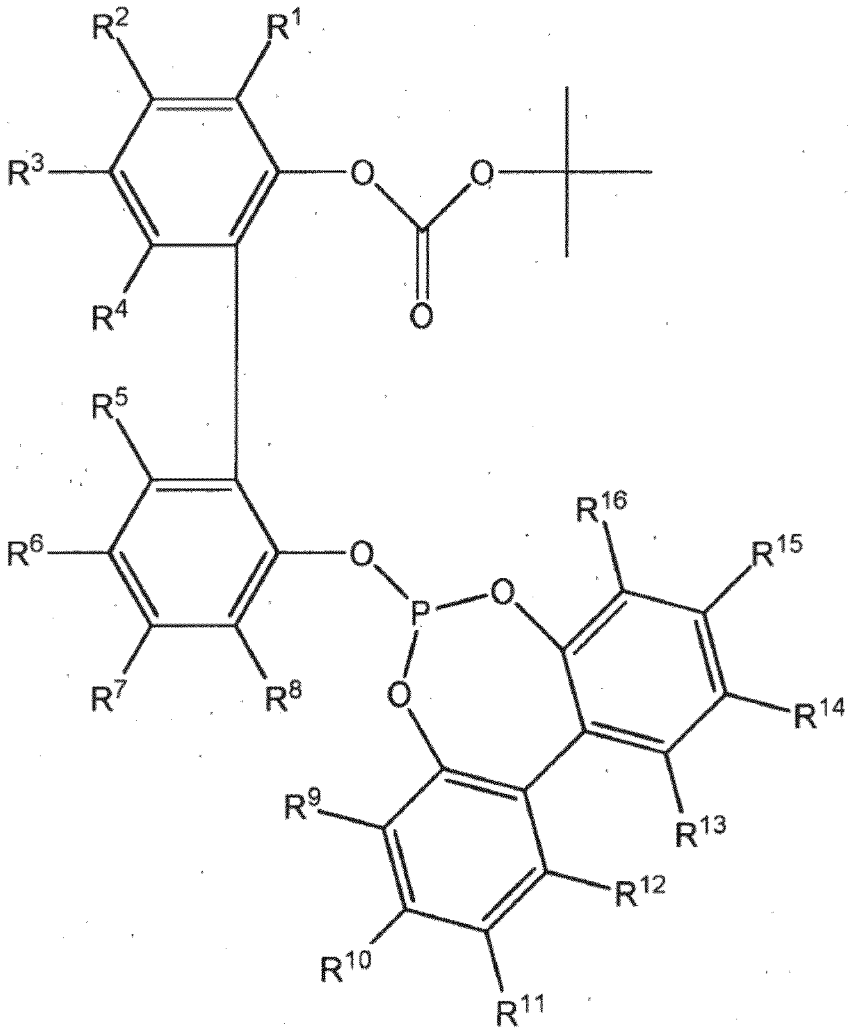

III

dans laquelle $R^9$, $R^{10}$, $R^{11}$, $R^{12}$, $R^{13}$, $R^{14}$, $R^{15}$, $R^{16}$ sont chacun choisis indépendamment les uns des autres parmi : : H, -alkyle en ($C_1$-$C_{12}$), -O-alkyle en ($C_1$-$C_{12}$), -0-aryle en ($C_6$-$C_{20}$), -aryle en ($C_6$-$C_{20}$), halogène, COO-alkyle en ($C_1$-$C_{12}$), CONH-alkyle en ($C_1$-$C_{12}$), -aryle en ($C_6$-$C_{20}$)-CON [alkyle en ($C_1$-$C_{12}$)]$_2$, -CO-alkyle en ($C_1$-$C_{12}$), -CO-aryle en ($C_6$-$C_{20}$), -COOH, -OH, $-SO_3H$ ; $-SO_3Na$, $-NO_2$, -CN, $-NH_2$, -N [alkyle en ($C_1$-$C_{12}$)]$_2$.

**12.** Ligand selon l'une quelconque des revendications 1 à 11, qui présente les structures générale IV :

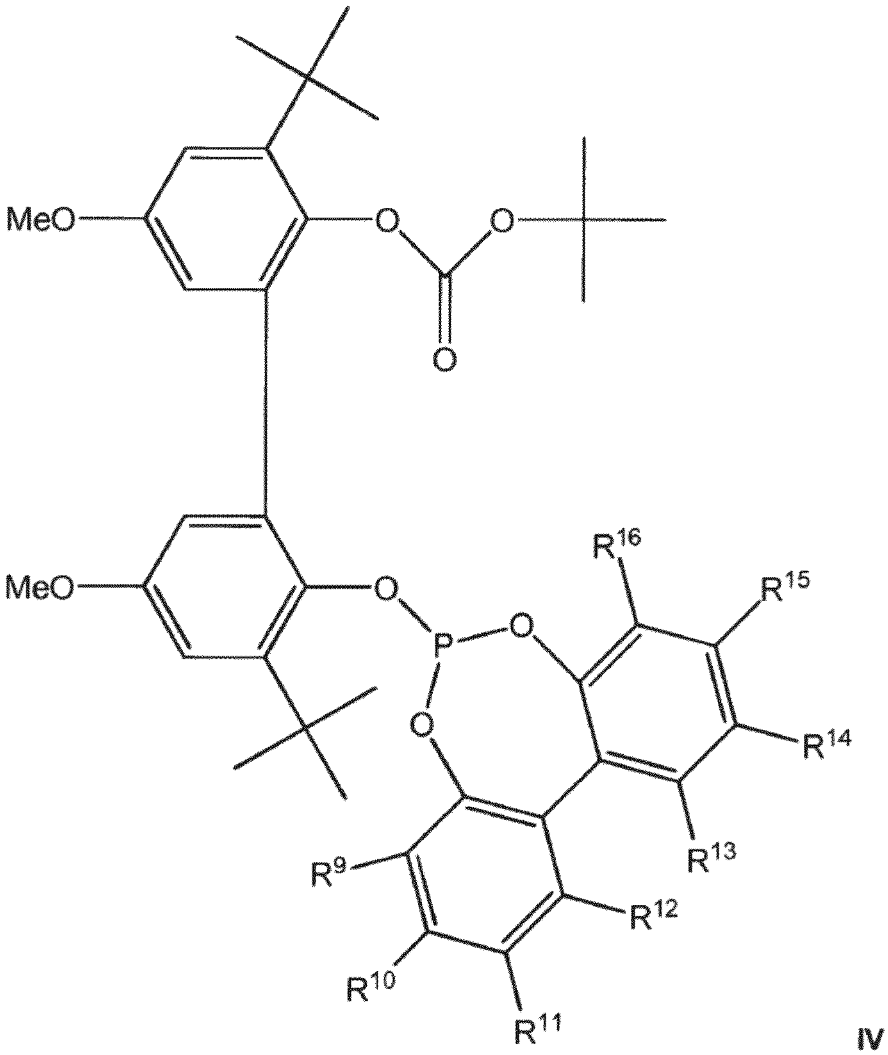

IV

dans laquelle $R^9$, $R^{10}$, $R^{11}$, $R^{12}$, $R^{13}$, $R^{14}$, $R^{15}$, $R^{16}$ sont chacun choisis indépendamment les uns des autres parmi : : H, -alkyle en ($C_1$-$C_{12}$), -O-alkyle en ($C_1$-$C_{12}$), -0-aryle en ($C_6$-$C_{20}$), -aryle en ($C_6$-$C_{20}$), halogène, -COO-alkyle en ($C_1$-$C_{12}$), CONH-alkyle en ($C_1$-$C_{12}$), -aryle en ($C_6$-$C_{20}$)-CON [alkyle en ($C_1$-$C_{12}$)]$_2$, -CO-alkyle en ($C_1$-$C_{12}$), -CO-aryle en ($C_6$-$C_{20}$), -COOH, -OH, -$SO_3H$ ; -$SO_3Na$, -$NO_2$, -CN, -$NH_2$, -N [alkyle en ($C_1$-$C_{12}$)]$_2$.

**13.** Complexe comprenant :

- un ligand selon l'une quelconque des revendications 1 à 12,
- un atome métallique choisi parmi : Rh, Ru, Co, Ir.

**14.** Utilisation d'un ligand selon l'une quelconque des revendications 1 à 12 dans un complexe ligand-métal pour la catalyse d'une réaction d'hydroformylation.

**15.** Procédé comprenant les étapes de procédé suivantes :

a) le chargement d'une oléfine,
b) l'ajout d'un complexe selon la revendication 13 ou d'un ligand selon l'une quelconque des revendications 1 à 12 et d'un composé qui comprend un atome métallique choisi parmi : Rh, Ru, Co, Ir,
c) l'introduction d' $H_2$ et de CO,
d) le chauffage du mélange réactionnel, l'oléfine étant transformée en un aldéhyde.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19954721
- EP 0155508 A


### In der Beschreibung aufgeführte Nicht-Patentliteratur


- **B. CORNILS ; W. A. HERRMANN.** Applied Homogeneous Catalysis with Organometallic Compounds. VCH, Weinheim, 1996, vol. 1 & 2
- **R. FRANKE ; D. SELENT ; A. BÖRNER.** Applied Hydroformylation. *Chem. Rev.,* 2012
- Angew. Chem. Int. Ed. 2000, vol. 39, 1639-1641
- **LEEUWEN.** *Journal of Catalysis,* 2013, vol. 298, 198-205
- Applied Hydroformylation. **R. FRANKE ; D. SELENT ; A. BÖRNER.** Chem. Rev. 2012, vol. 112, 5681
- **SIEHE HIERZU R. FRANKE ; D. SELENT ; A. BÖRNER.** Applied Hydroformylation. *Chem. Rev.,* 2012
- *General Method for the Preparation of a P-Modified Rh precatalyst,* 5688
- **P. W. N. M. VAN LEEUWEN.** Rhodium Catalyzed Hydroformylation. 2000, S. 48 ff, , S.233 ff
- **K.D. WIESE ; D. OBST.** Top. Organomet. Chem. Springer Verlag Berlin Heidelberg, 2006, vol. 18, 1-13
- **W. L. F. ARMAREGO ; CHRISTINA CHAI ; BUTTERWORTH HEINEMANN.** Purification of Laboratory Chemicals. Elsevier, 2009
- **ROBIN K. HARRIS ; EDWIN D. BECKER ; SONIA M. CABRAL DE MENEZES ; ROBIN GOODFELLOW ; PIERRE GRANGER.** *Pure Appl. Chem.,* 2001, vol. 73, 1795-1818
- **ROBIN K. HARRIS ; EDWIN D. BECKER ; SONIA M. CABRAL DE MENEZES ; PIERRE GRANGER ; ROY E. HOFFMAN ; KURT W. ZILM.** *Pure Appl. Chem.,* 2008, vol. 80, 59-84